# EUROPEAN PATENT APPLICATION

(11) **EP 4 699 605 A1**
(43) Date of publication of application: **25.02.2026**
(21) Application number: 24811338.3
(22) Date of filing: 16.05.2024
(51) Int. Cl.: A61K 31/423, A61P 25/00

(54) **USE OF NOVEL COMPOUND FOR TREATING INTRACTABLE DISEASES**

(30) Priority: 19.05.2023 KR 20230064957
(71) Applicant: YJ Cerapeutics Inc., Seoul 01811 (KR)
(72) Inventor: YUNE, Tae Young, Seoul 01801 (KR); LEE, Jee Youn, Seoul 01721 (KR)
(74) Representative: Isarpatent
(86) International application number: PCT/KR2024/006626
(87) International publication number: WO 2024/242399

(57) **Abstract**

The present disclosure relates to: a composition of a novel compound YJ102 for preventing or treating bladder diseases; a composition for preventing or treating neuropathic pain, neuroinflammation or inflammatory skin diseases; or use of the compound for treating intractable nervous system diseases including neurogenic bladder. The compound YJ102 of the present disclosure inhibits neuropathic pain caused by various causes with an effect superior to those of conventional drugs, alleviates urinary disturbances caused by spinal cord injury, restores bladder hypertrophy and bladder functions, inhibits redness and keratinization, which are major symptoms of psoriasis in a psoriasis animal model, inhibits an increase in epidermal layer thickness, and inhibits spleen hypertrophy caused by psoriasis, and thus can be utilized for treatment of various intractable nervous system diseases or bladder diseases.

## Description

### [Technical Field]

The present disclosure relates to a composition of a novel compound YJ102 for preventing or treating neuropathic pain, neuroinflammation, bladder diseases or inflammatory skin diseases, or to a use of the compound for the treatment of intractable nervous system diseases including neurogenic bladder.

### [Background Art]

Pain is defined as an unpleasant sensory and emotional experience associated with actual or potential tissue damage, or described in terms of such damage. The pain also refers to nociception and sensory disorders that occur by stimulating the area that contacts the cerebral cortex and the limbic system area through nerve tracts consisting of nociceptors and nerve fibers. The pain may be referred to as an alarm reaction that delivers abnormalities inside or outside the body as a defense mechanism to protect the body. Since the pain itself is not a disease, even if the pain is removed, diseases causing the pain are not cured. The causes may be broadly divided into perceptual causes caused by damage or inflammation of somatic or visceral tissues, and neuropathic causes caused by nerve injury. In the case of the perceptual cause, pain may include cutaneous pain, visceral pain, somatic pain, perceptual neuralgia, radicular referred pain, and somatic referred pain, and in the case of the neuropathic cause, pain may be caused by peripheral or central nervous dysfunction. The neuropathic pain is pain caused by damage or dysfunction of the nervous system, and is caused by abnormalities in the transmission or processing of pain signals in the nervous system. The neuropathic pain may be caused by pressure, entrapment, injury and infection of nerves, diseases such as diabetes or multiple sclerosis, specific drugs, or the like. The pain is expressed as burning or stabbing sensation, or the like, and may be accompanied by numbness or paralysis of the affected area. Since the neuropathic pain is most often diagnosed based on symptoms, any pain that is typically characterized by burning and/or shooting pain and/or numbness and/or tingling and/or allodynia is considered neuropathic. Other features of the neuropathic pain include hyperalgesia (significantly excessive pain sensation to stimuli), hyperesthesia (increased sensitivity to normal stimuli), dysesthesia (unpleasant abnormal sensations when no injury has occurred), and tingling (abnormal sensations such as "tingling feeling" either spontaneous or provoked). Glial cells, which are non-neuronal cells in the nervous system, are known to be involved in the occurrence and maintenance of neuropathic pain. In particular, within the spinal cord, microglia and astrocytes play a key role in the pathogenesis of neuropathic pain. After nerve injury, the microglia and astrocytes are activated to secrete pro-inflammatory cytokines and chemokines that cause inflammatory responses. As a result, the inflammation and damage to nerve fibers are further deepened. In addition, the glial cells also contribute to occurrence of central sensitization, a phenomenon in which neurons in the spinal cord become oversensitive to pain signals. Accordingly, treatments targeting glial cells in the spinal cord are emerging as a potential alternative for treating neuropathic pain. The central sensitization refers to a phenomenon in which pain persists due to changes in the spinal nervous system. Pain stimuli occurring in the central nervous system are primarily processed in the spinal dorsal horn.

Neurons constitute the nervous system and are essential for controlling sensory perception, learning, memory, and motor skills. However, once damaged, it take a long time to recover the damaged neurons to normal, and unlike other cells, the neurons are difficult to regenerate, so that there is a high possibility of permanent damage. In this regard, even in the same neurons, there are differences in damage response and regenerative ability depending on which part has been damaged. In the case of spinal cord injury (SCI), which is a central nervous system, regeneration failure occurs more frequently than in other parts, and not only medical disorders such as motor dysfunction and urinary dysfunction, but also psychological disorders are accompanied. The bladder is a hollow sac-like muscular organ responsible for storing and excreting urine, and is located in the pelvis and behind the pubic symphysis (symphysis pubica), and is connected to the urethra below the bladder and the ureters above the bladder, and in men, the prostate is connected to the lower part of the bladder. The bladder stores up to 400 to 500 cc of urine and discharges the stored urine through the urethra according to nerve signals. When the bladder functions are damaged, various bladder diseases are caused. The causes of these damaged bladder functions may be broadly divided into two types. One cause is dysfunction caused by external infection, and the other cause is dysfunction caused by physical abnormality. Among these, the dysfunction caused by physical abnormality may be divided into dysfunctions caused by abnormalities in the bladder itself and nerve injury. A representative disease caused by bladder dysfunction due to nerve injury is overactive bladder syndrome, which refers to a disease in which the bladder functions are too sensitive due to psychological or pathological factors in a patient, so that the bladder muscles contract abnormally and frequently while storing urine in the bladder, causing the patient to feel an urgent desire to urinate and urinate frequently. Common symptoms of the overactive bladder syndrome include urinary frequency of frequently urinating at least 8 times a day, urinary urgency of being hard to bear when going to pee once, nocturia of waking up more than twice at night to urinate, urgency incontinence of leakage of urine with a suddenly uncontrollable urge to urinate, and the like. Neurogenic bladder, known as a type of overactive bladder syndrome, refers to a disease that exhibits bladder and urethral dysfunction caused by damage to the nervous system from various causes such as diabetes, cerebrovascular accident, brain injury, nerve injury, or spinal cord injury (SCI). The neurogenic bladder may accompany several complications, mainly including urinary tract infections, bladder and kidney damage, and bladder stones. When damage to the nervous system causes abnormalities in the central nervous system related to the urinary tract, the nerve signals connected to the urinary tract are transmitted abnormally, and thus it is known that the neurogenic bladder is induced. Since such neurogenic bladder is essentially a derived disease caused by a nerve disease, it is known that the overactive bladder syndrome relapses within a short period of time even when treated with known methods, and thus the development of a treatment method therefor has been required.

Meanwhile, the human immune system serves to protect the body from foreign antigens that invade the body, but does not attack its own tissues due to self-tolerance. However, when the self-tolerance of the immune system is destroyed and immune cells recognize proteins normally expressed by own genes as targets for attack to create antibodies or trigger T-cell responses to destroy normal tissues, which is called autoimmunity, and when specific symptoms appear, it is called an autoimmune disease. The inflammatory response, an immune response, is a repair mechanism that attempts to regenerate damaged areas caused by chemical substances, bacterial infections, and physical actions that bring about organic changes in the body or tissue. When inflammation is expressed by harmful stimuli, infections, and trauma, vasoactive substances such as inflammatory ingredients are released locally to cause inflammation. If such an inflammatory response persists, mucosal damage is rather promoted to cause inflammatory diseases such as rheumatoid arthritis, arteriosclerosis, gastritis, and asthma, which are caused by redness, fever, swelling, pain, and functional disorders. Psoriasis, one of the inflammatory diseases, is a non-contagious chronic inflammatory skin disease that occurs in skin tissue due to an excessive increase in the immune response of abnormally activated T cells, and an intractable immune disease to be overcome as an autoimmune disease. When the psoriasis develops, the psoriasis often progresses to a other organ diseases and may be accompanied by various diseases such as psoriatic arthritis, metabolic syndrome, and cardiovascular diseases. The number of psoriasis patients reaches approximately 2 to 3% of the world's population, and in Korea, the number has increased by approximately 8,500 over the past five years since 2012, which has been estimated as 168,000, reaching approximately 3% of the population. The psoriasis is a disease in which erythematous papules and plaques with distinct borders and covered with silvery scales of various sizes are formed on the skin. The histological characteristic of the psoriasis is hyperproliferation of the epithelium, and also shows various clinical features, such as pus, scales, and rash. The cause of psoriasis is not yet clearly known, but factors suspected to be the cause include genetic factors, environmental aggravation or triggers, immunological factors, and the like. Currently, it is known that individual lifestyle and environmental factors act as causative factors based on genetic factors, and that immunological factors cause proliferation of keratinocytes and inflammatory responses. Factors of worsening or causing psoriasis include skin trauma, infections, cold and dry climates such as winter, dry skin, stress, and drugs. Treatment methods for psoriasis include applying drugs to the affected area or phototherapy using ultraviolet rays. The drugs are used with drugs, such as synthetic vitamin A derivatives, immunosuppressants such as cyclosporine, methotrexate, and steroids, and the like, including adrenal cortex hormones, vitamin D derivatives, moisturizers. However, if strong adrenal cortex hormones are applied for too long, various side effects such as capillary dilation, striae dilatation, and skin atrophy may occur, cyclosporine may cause kidney dysfunction and blood pressure problems, and methotrexate has the problem of causing cirrhosis or fibrosis if used cumulatively.

### [Disclosure]

### [Technical Problem]

An object of the present disclosure is to provide a pharmaceutical composition for preventing or treating neuropathic pain.

In addition, another object of the present disclosure is to provide a pharmaceutical composition for preventing or treating neuroinflammation.

In addition, yet another object of the present disclosure is to provide a pharmaceutical composition for preventing or treating bladder diseases.

In addition, still another object of the present disclosure is to provide a pharmaceutical composition for preventing or treating neurogenic bladder.

In addition, another object of the present disclosure is to provide a composition for alleviating bladder dysfunction.

In addition, another object of the present disclosure is to provide a composition for inhibiting bladder hypertrophy.

In addition, another object of the present disclosure is to provide a pharmaceutical composition for preventing or treating inflammatory skin diseases.

In addition, still another object of the present disclosure is to provide a cosmetic composition for preventing or alleviating inflammatory skin diseases.

In addition, another object of the present disclosure is to provide a use for the preparation of a pharmaceutical composition for preventing or treating neuropathic pain, neuroinflammation, bladder diseases, neurogenic bladder or inflammatory skin diseases.

In addition, another object of the present disclosure is to provide a use for the preparation of a composition for alleviating bladder dysfunction or inhibiting bladder hypertrophy.

In addition, still another object of the present disclosure is to provide a method for treating neuropathic pain, neuroinflammation, bladder diseases, neurogenic bladder or inflammatory skin diseases.

In addition, another object of the present disclosure is to provide a method for treating or alleviating bladder dysfunction or bladder hypertrophy.

### [Technical Solution]

In order to achieve the object, an aspect of the present disclosure provides a pharmaceutical composition for preventing or treating neuropathic pain including a compound represented by Chemical Formula 1.

In addition, another aspect of the present disclosure provides a pharmaceutical composition for preventing or treating neuroinflammation including a compound represented by Chemical Formula 1.

In addition, yet another aspect of the present disclosure provides a pharmaceutical composition for preventing or treating bladder diseases including a compound represented by Chemical Formula 1.

In addition, still another aspect of the present disclosure provides a pharmaceutical composition for preventing or treating neurogenic bladder including a compound represented by Chemical Formula 1.

In addition, still another aspect of the present disclosure provides a composition for alleviating bladder dysfunction including a compound represented by Chemical Formula 1.

In addition, still another aspect of the present disclosure provides a composition for inhibiting bladder hypertrophy including a compound represented by Chemical Formula 1.

In addition, still another aspect of the present disclosure provides a pharmaceutical composition for preventing or treating inflammatory skin diseases including a compound represented by Chemical Formula 1.

In addition, still another aspect of the present disclosure provides a cosmetic composition for preventing or alleviating inflammatory skin diseases including a compound represented by Chemical Formula 1.

In addition, still another aspect of the present disclosure provides a use of a compound represented by Chemical Formula 1 for the preparation of a pharmaceutical composition for preventing or treating neuropathic pain, neuroinflammation, bladder diseases, neurogenic bladder or inflammatory skin diseases.

**In** addition, still another aspect of the present disclosure provides a use of a compound represented by Chemical Formula 1 for the preparation of a composition for alleviating bladder dysfunction or inhibiting bladder hypertrophy.

**In** addition, still another aspect of the present disclosure provides a method for treating neuropathic pain, neuroinflammation, bladder diseases, neurogenic bladder or inflammatory skin diseases.

**In** addition, still another aspect of the present disclosure provides a method for treating or alleviating bladder dysfunction or bladder hypertrophy by a compound represented by Chemical Formula 1.

### [Advantageous Effects]

According to the present disclosure, the compound YJ102 of the present disclosure inhibits neuropathic pain caused by various causes with an effect superior to those of conventional drugs, particularly, exhibits an effect of inhibiting neuroinflammation caused by nerve injury, alleviates urinary disturbances caused by spinal cord injury by inhibiting urothelium death, tight junction disruption, cell infiltration, and bladder inflammation, restores bladder hypertrophy and bladder functions, inhibits redness and keratinization, which are major symptoms of psoriasis in a psoriasis animal model, inhibits an increase in epidermal layer thickness, and inhibits spleen hypertrophy caused by psoriasis, and thus can be utilized for the prevention or treatment of neuropathic pain or neuroinflammation, the prevention or treatment of bladder diseases, neurogenic bladder or bladder dysfunction, and the treatment of inflammatory skin diseases caused by inflammation such as psoriasis.

### [Description of Drawings]

FIG. 1A is a diagram showing a process of preparing an SCI animal model.
FIG. 1B is a diagram showing a process of preparing an LSS animal model.
FIG. 1C is a diagram showing a process of preparing an SNI animal model.
FIG. 1D is a diagram showing results of measuring mechanical allodynia in an SCI animal model after oral administration of YJ102.
FIG. 1E is a diagram showing results of measuring mechanical allodynia in an LSS animal model after oral administration of YJ102.
FIG. 1F is a diagram showing results of measuring mechanical allodynia in an SNI animal model after oral administration of YJ102.
FIG. 1G is a diagram showing results of measuring mechanical allodynia in an SCI animal model after intraperitoneal administration of YJ102, gabapentin, or pregabalin.
FIG. 1H is a diagram showing results of measuring mechanical allodynia in an LSS animal model after intraperitoneal administration of YJ102, gabapentin, or pregabalin.
FIG. 1I is a diagram showing results of measuring mechanical allodynia in an SNI animal model after intraperitoneal administration of YJ102, gabapentin, or pregabalin.
FIG. 2A is a diagram showing results of analyzing the activation of microglia in an SNI animal model by immunohistochemistry:
   Cont: contralateral tissue; and
   Ipsi: Ipsilateral tissue.
FIG. 2B is a diagram showing results of analyzing the activation of microglia in an SNI animal model by immunofluorescence chemistry:
   Cont: contralateral tissue; and
   Ipsi: Ipsilateral tissue.
FIG. 2C is a diagram showing results of analyzing the activation of microglia in an SNI animal model by Western blot:
   Cont: contralateral tissue; and
   Ipsi: Ipsilateral tissue.
FIG. 3A is a diagram showing results of analyzing the activation of astrocytes in an SNI animal model by immunohistochemistry:
   Cont: contralateral tissue; and
   Ipsi: Ipsilateral tissue.
FIG. 3B is a diagram showing results of analyzing the activation of astrocytes in an SNI animal model by Western blot:
   Cont: contralateral tissue; and
   Ipsi: Ipsilateral tissue.
FIG. 4A is a diagram showing results of analyzing the expression of inflammatory factors in an SNI animal model by RT-PCR.
FIG. 4B is a diagram showing results of analyzing the expression of inflammatory factors in an SNI animal model by Western blot analysis.
FIG. 5A is a diagram showing results of analyzing the expression of factors associated with a JAK/STAT3 signaling pathway in an inflammation-inhibiting mechanism in an SNI animal model by Western blot.
FIG. 5B is a diagram showing results of analyzing the expression of factors associated with a mTOR signaling pathway in an inflammation-inhibiting mechanism in an SNI animal model by Western blot.
FIG. 5C is a diagram showing results of analyzing the expression of TRMP7 in an SNI animal model by Western blot.
FIG. 5D is a diagram showing results of analyzing the expression and activation of JMJD3 in an SNI animal model by Western blot.
FIG. 6A is a diagram showing a bladder function improvement effect of YJ102 in a spinal cord injury model by measuring a residual urine volume:
   Sham: Group subjected to only laminectomy without damage;
   Veh: Control group (group administered with a solution of DMSO, methylpyrrolidinone, and water); and
   YJ102: YJ102 administered group.
FIG. 6B is a diagram showing a bladder function improvement effect of YJ102 in a spinal cord injury model using a bladder function score:
   Sham: Group subjected to only laminectomy without damage;
   Veh: Control group (group administered with a solution of DMSO, methylpyrrolidinone, and water); and
   YJ102: YJ102 administered group.
FIG. 6C is a diagram showing a bladder function improvement effect of YJ102 in a spinal cord injury model by the size of an extracted bladder:
   Sham: Group subjected to only laminectomy without damage;
   Veh: Control group (group administered with a solution of DMSO, methylpyrrolidinone, and water); and
   YJ102: YJ102 administered group.
FIG. 6D is a diagram showing a bladder function improvement effect of YJ102 in a spinal cord injury model by a bladder weight:
   Sham: Group subjected to only laminectomy without damage;
   Veh: Control group (group administered with a solution of DMSO, methylpyrrolidinone, and water); and
   YJ102: YJ102 administered group.
FIG. 7A is a diagram showing bladder hypertrophy in a spinal cord injury model by H&E staining:
   SCI 1d: Day 1 after spinal cord injury;
   Sham: Group subjected to only laminectomy without damage;
   Veh: Control group (group administered with a solution of DMSO, methylpyrrolidinone, and water); and
   YJ102: YJ102 administered group.
FIG. 7B is a diagram showing the thicknesses of a urothelium layer, a lamina propria, and a muscle layer in a spinal cord injury model.
FIG. 7C is a diagram showing a collagen content in the bladder muscle in a spinal cord injury model by Masson's trichrome staining (Blue: collagen):
   SCI 1d: Day 1 after spinal cord injury;
   Sham: Group subjected to only laminectomy without damage;
   Veh: Control group (group administered with a solution of DMSO, methylpyrrolidinone, and water); and
   YJ102: YJ102 administered group.
FIG. 7D is a diagram showing a collagen/muscle ratio (%) in the bladder in a spinal cord injury model:
   SCI 1d: Day 1 after spinal cord injury;
   Sham: Group subjected to only laminectomy without damage;
   Veh: Control group (group administered with a solution of DMSO, methylpyrrolidinone, and water); and
   YJ102: YJ102 administered group.
FIG. 8A is a diagram showing the expression of ZO-1 and Occludin, tight junction proteins of the bladder urothelium, in a spinal cord injury model by Western blot analysis:
   SCI 1d: Day 1 after spinal cord injury;
   Sham: Group subjected to only laminectomy without damage;
   Veh: Control group (group administered with a solution of DMSO, methylpyrrolidinone, and water); and
   YJ102: YJ102 administered group.
FIG. 8B is a diagram showing immunofluorescence staining results of Occludin (Red) in the urothelium layer in a spinal cord injury model:
   Sham: Group subjected to only laminectomy without damage;
   Veh: Control group (group administered with a solution of DMSO, methylpyrrolidinone, and water); and
   YJ102: YJ102 administered group.
FIG. 9A is a diagram showing introduction of MPO (neutrophils) (Green) into the bladder on day 1 after spinal cord injury in a spinal cord injury model by immunofluorescence staining:
   SCI 1d: Day 1 after spinal cord injury;
   Sham: Group subjected to only laminectomy without damage;
   Veh: Control group (group administered with a solution of DMSO, methylpyrrolidinone, and water); and
   YJ102: YJ102 administered group.
FIG. 9B is a graph showing the relative fluorescence intensity of MPO-positive cells in the bladder on day 1 after spinal cord injury in a spinal cord injury model:
   Sham: Group subjected to only laminectomy without damage;
   Veh: Control group (group administered with a solution of DMSO, methylpyrrolidinone, and water); and
   YJ102: YJ102 administered group.
FIG. 9C is a diagram showing introduction of ED-1 (macrophage) (Green) into the bladder on day 7 after spinal cord injury in a spinal cord injury model by immunofluorescence staining:
   SCI 7d: Day 7 after spinal cord injury;
   Sham: Group subjected to only laminectomy without damage;
   Veh: Control group (group administered with a solution of DMSO, methylpyrrolidinone, and water); and
   YJ102: YJ102 administered group.
FIG. 9D is a graph showing the relative fluorescence intensity of ED-1 positive cells in the bladder on day 7 after spinal cord injury in a spinal cord injury model:
   Sham: Group subjected to only laminectomy without damage;
   Veh: Control group (group administered with a solution of DMSO, methylpyrrolidinone, and water); and
   YJ102: YJ102 administered group.
FIG. 9E is a diagram showing Western blot analysis results of ED-1 in the bladder on day 7 after spinal cord injury in a spinal cord injury model:
   Sham: Group subjected to only laminectomy without damage;
   Veh: Control group (group administered with a solution of DMSO, methylpyrrolidinone, and water); and
   YJ102: YJ102 administered group.
FIG. 10A is a diagram showing TUNEL (Blue) staining results in the urothelium layer on day 3 after spinal cord injury in a spinal cord injury model:
   Sham: Group subjected to only laminectomy without damage;
   Veh: Control group (group administered with a solution of DMSO, methylpyrrolidinone, and water); and
   YJ102: YJ102 administered group.
FIG. 10B is a graph showing the number of TUNEL-positive cells in the urothelium layer on day 3 after spinal cord injury in a spinal cord injury model:
   Sham: Group subjected to only laminectomy without damage;
   Veh: Control group (group administered with a solution of DMSO, methylpyrrolidinone, and water); and
   YJ102: YJ102 administered group.
FIG. 10C is a diagram showing Western blot analysis results of cleaved caspase-3 (activated caspase-3) in the urothelium layer on day 3 after spinal cord injury in a spinal cord injury model:
   Sham: Group subjected to only laminectomy without damage;
   Veh: Control group (group administered with a solution of DMSO, methylpyrrolidinone, and water); and
   YJ102: YJ102 administered group.
FIG. 10D is a diagram showing Western blot analysis results of UPIII (urothelium maker) in the urothelium layer on day 3 after spinal cord injury in a spinal cord injury model:
   Sham: Group subjected to only laminectomy without damage;
   Veh: Control group (group administered with a solution of DMSO, methylpyrrolidinone, and water); and
   YJ102: YJ102 administered group.
FIG. 11A is a diagram showing the expression of p75 in the urothelium layer on days 1, 3, and 7 after spinal cord injury in a spinal cord injury model by RT-PCR analysis:
   Sham: Group subjected to only laminectomy without damage;
   1d: Day 1 after spinal cord injury;
   3d: Day 3 after spinal cord injury; and
   7d: Day 7 after spinal cord injury.
FIG. 11B is a diagram showing the expression of p75 in the urothelium layer on days 1, 3, and 7 after spinal cord injury in a spinal cord injury model by Western blot analysis:
   Sham: Group subjected to only laminectomy without damage;
   1d: Day 1 after spinal cord injury;
   3d: Day 3 after spinal cord injury; and
   7d: Day 7 after spinal cord injury.
FIG. 11C is a diagram showing the expression of p75 in the urothelium layer on days 1 and 7 after spinal cord injury in a spinal cord injury model by immunofluorescence staining:
   Sham: Group subjected to only laminectomy without damage;
   1d: Day 1 after spinal cord injury; and
   7d: Day 7 after spinal cord injury.
FIG. 11D is a diagram showing results of identifying and counting p75 (Red)/TUNEL (Green) double-expressing cells by immunofluorescence staining:
   Sham: Group subjected to only laminectomy without damage;
   1d: Day 1 after spinal cord injury;
   7d: Day 7 after spinal cord injury.
   U: urothelium;
   L: lamina propria;
   M: muscle layer; and
   Arrow: p75/TUNEL double-positive cells.
FIG. 11E is a diagram showing a p75-mediated urothelial apoptosis inhibitory effect of YJ102 in a spinal cord injury model by RT-PCR analysis of p75:
   1d: Day 1 after spinal cord injury;
   7d: Day 7 after spinal cord injury.
   Sham: Group subjected to only laminectomy without damage;
   Veh: Control group (group administered with a solution of DMSO, methylpyrrolidinone, and water); and
   YJ102: YJ102 administered group.
FIG. 11F is a diagram showing a p75-mediated urothelial apoptosis inhibitory effect of YJ102 in a spinal cord injury model by Western blot analysis:
   1d: Day 1 after spinal cord injury;
   7d: Day 7 after spinal cord injury.
   Sham: Group subjected to only laminectomy without damage;
   Veh: Control group (group administered with a solution of DMSO, methylpyrrolidinone, and water); and
   YJ102: YJ102 administered group.
FIGS. 12A and 12C are diagrams showing the expression of NGF in macrophages in a spinal cord injury model by RT-PCR analysis:
   Sham: Group subjected to only laminectomy without damage;
   1d: Day 1 after spinal cord injury;
   3d: Day 3 after spinal cord injury; and
   7d: Day 7 after spinal cord injury.
FIGS. 12B and 12D are diagrams showing the expression of NGF and proNGF in macrophages in a spinal cord injury model by Western blot analysis:
   Sham: Group subjected to only laminectomy without damage;
   1d: Day 1 after spinal cord injury;
   3d: Day 3 after spinal cord injury; and
   7d: Day 7 after spinal cord injury.
FIG. 12E is a diagram showing proNGF (Red) and ED-1 (Green) double-positive cells in macrophages in a spinal cord injury model by immunofluorescence staining:
   Sham: Group subjected to only laminectomy without damage;
   SCI (7 d): Day 7 after spinal cord injury.
FIGS. 13A and 13C are diagrams showing the expression of NGF after inflammation induction (LPS treatment) in macrophage cell lines by RT-PCR:
   2 h: 2 hours after LPS treatment;
   6 h: 6 hours after LPS treatment; and
   12 h: 12 hours after LPS treatment.
FIGS. 13B and 13D are diagrams showing the expression of NGF and proNGF after inflammation induction (LPS treatment) in macrophage cell lines by Western blot analysis:
   2 h: 2 hours after LPS treatment;
   6 h: 6 hours after LPS treatment; and
   12 h: 12 hours after LPS treatment.
FIG. 14A is a diagram showing the expression of inflammatory cytokine and chemokine in the bladder in a spinal cord injury model by RT-PCR analysis:
   Sham: Group subjected to only laminectomy without damage;
   Veh: Control group (group administered with a solution of DMSO, methylpyrrolidinone, and water); and
   YJ102: YJ102 administered group.
   1d: Day 1 after spinal cord injury;
   3d: Day 3 after spinal cord injury; and
   7d: Day 7 after spinal cord injury.

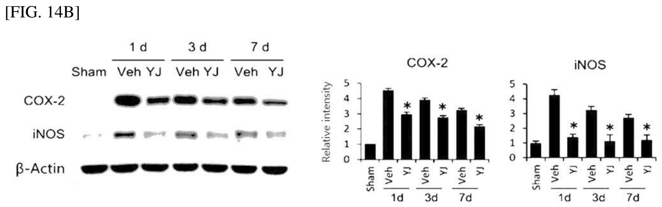

FIG. 14B is a diagram showing the expression of inflammatory factors in the bladder in a spinal cord injury model by Western blot analysis:
   Sham: Group subjected to only laminectomy without damage;
   Veh: Control group (group administered with a solution of DMSO, methylpyrrolidinone, and water); and
   YJ: YJ102 administered group.
   1d: Day 1 after spinal cord injury;
   3d: Day 3 after spinal cord injury; and
   7d: Day 7 after spinal cord injury.

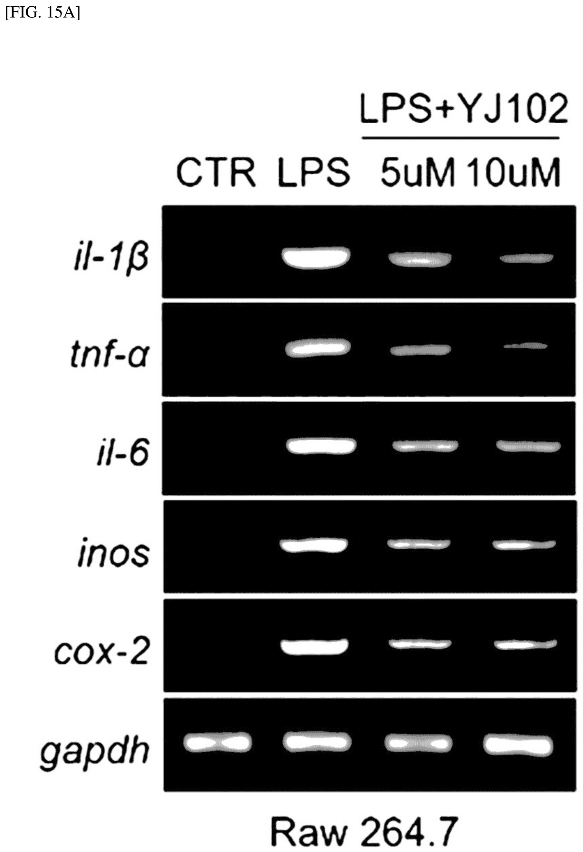

FIG. 15A is a diagram showing the expression of inflammatory factors after inflammation induction (LPS treatment) in a macrophage cell line by RT-PCR analysis.

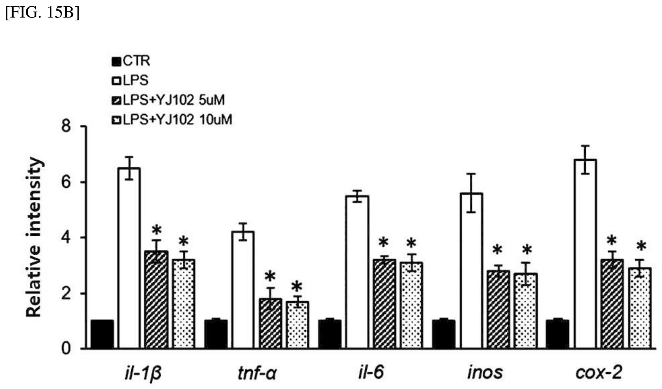

FIG. 15B is a graph showing the quantification of the results of 15A.

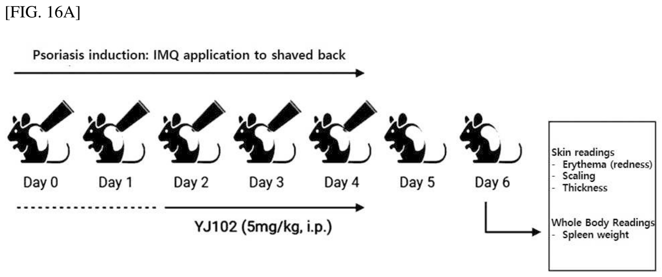

FIG. 16A is a schematic diagram showing a study on the efficacy evaluation of YJ102 using an IMQ animal model as a psoriasis animal model.

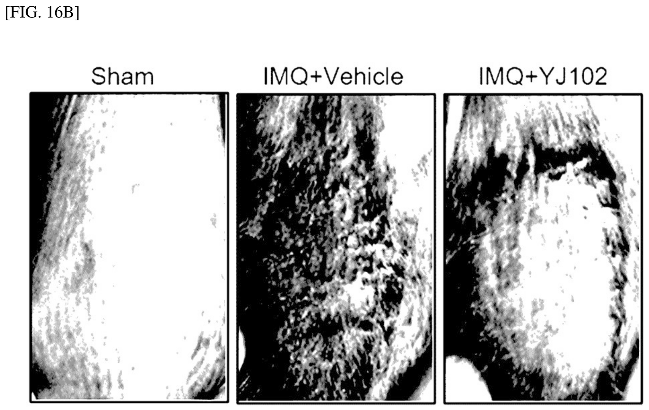

FIG. 16B is a diagram showing back images of IMQ animal model mice on Day 5:
   Sham: Control group applied with Vaseline cream;
   IMQ+Vehicle: Psoriasis animal model group prepared with IMQ; and
   IMQ+YJ102: Group of administering YJ102 to psoriasis animal model.

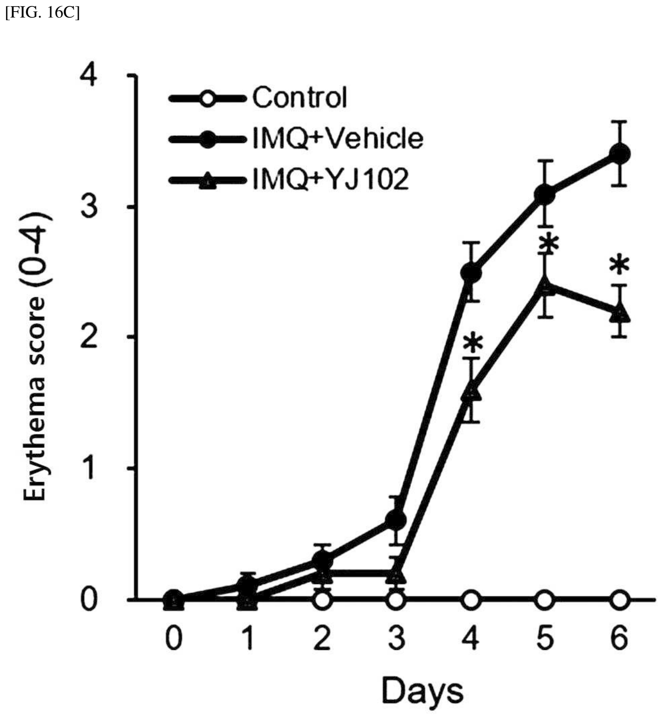

FIG. 16C is a diagram showing PASI scores of evaluating redness in a psoriasis animal model:
   ham: Control group applied with Vaseline cream;
   IMQ+Vehicle: Psoriasis animal model group prepared with IMQ; and
   IMQ+YJ102: Group of administering YJ102 to psoriasis animal model.

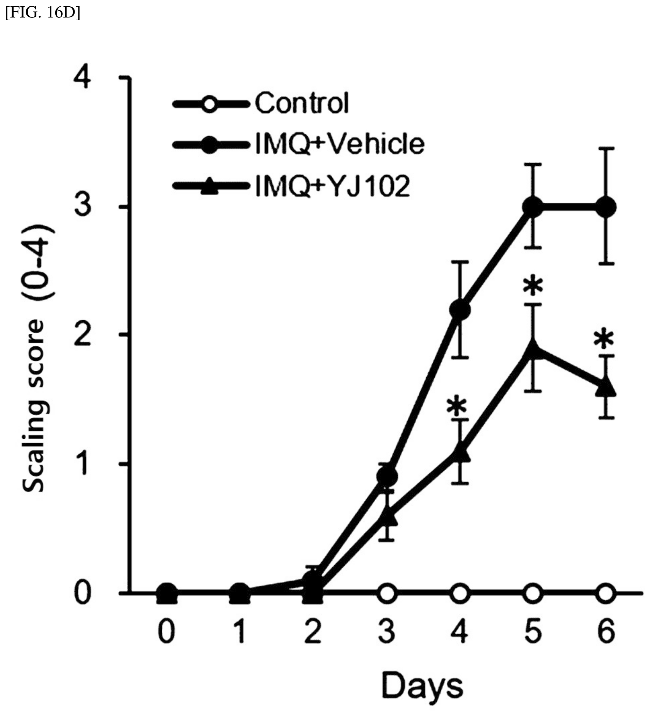

FIG. 16D is a diagram showing PASI scores of evaluating the degree of keratinization:
   ham: Control group applied with Vaseline cream;
   IMQ+Vehicle: Psoriasis animal model group prepared with IMQ; and
   IMQ+YJ102: Group of administering YJ102 to psoriasis animal model.

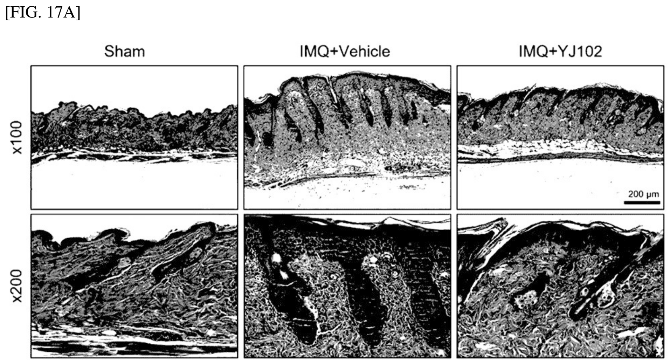

FIG. 17A is a diagram showing H&E staining results of skin tissue in a psoriasis animal model:
   Sham: Control group applied with Vaseline cream;
   IMQ+Vehicle: Psoriasis animal model group prepared with IMQ; and
   IMQ+YJ102: Group of administering YJ102 to psoriasis animal model.

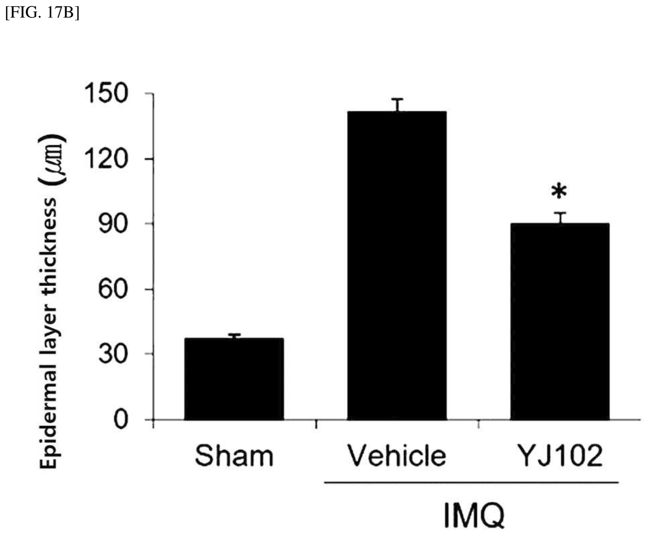

FIG. 17B is a graph showing the quantification of the epidermal layer thickness in a psoriasis animal model:
   Sham: Control group applied with Vaseline cream;
   IMQ+Vehicle: Psoriasis animal model group prepared with IMQ; and
   IMQ+YJ102: Group of administering YJ102 to psoriasis animal model.

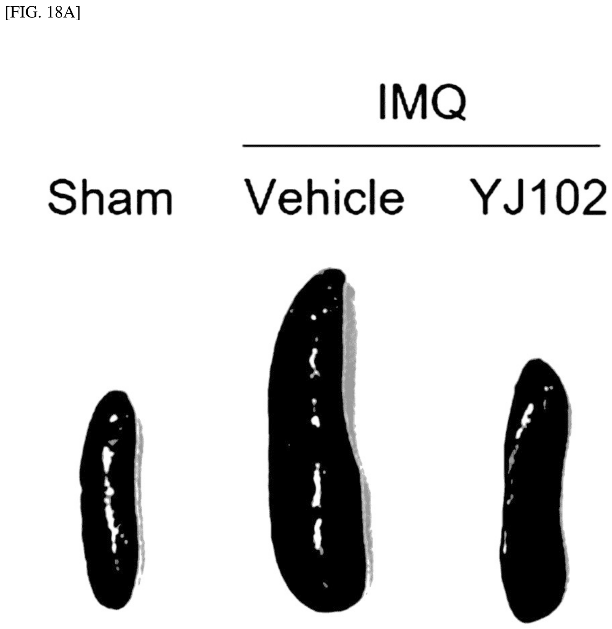

FIG. 18A is a diagram showing images of the spleen extracted from a psoriasis animal model on Day 6:
   Sham: Control group applied with Vaseline cream;
   Vehicle: Psoriasis animal model group prepared with IMQ; and
   YJ102: Group of administering YJ102 to psoriasis animal model.

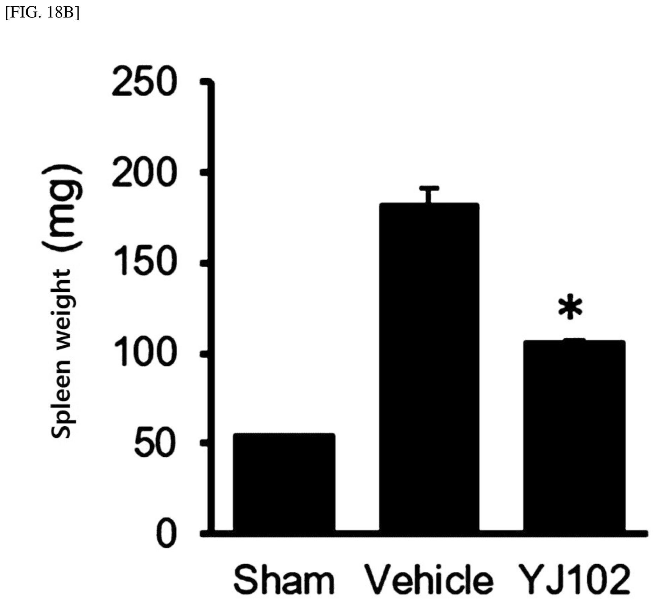

FIG. 18B is a diagram showing weights of the spleen extracted from a psoriasis animal model on Day 6:
   Sham: Control group applied with Vaseline cream;
   Vehicle: Psoriasis animal model group prepared with IMQ; and
   YJ102: Group of administering YJ102 to psoriasis animal model.

### [Best Mode]

Hereinafter, embodiments of the present disclosure will be described in detail with reference to the accompanying drawings. However, the following embodiments are presented as examples for the present disclosure, and when it is determined that the detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

Further, terminologies used in the present disclosure are a terminologies used to properly express embodiments of the present disclosure, which may vary according to a user, an operator's intention, or customs in the art to which the present disclosure pertains. Therefore, these terminologies used herein will be defined based on the contents throughout the specification. Throughout this specification, unless explicitly described to the contrary, when a certain part "comprises" a certain component, it will be understood to further include other components, not the exclusion of other components.

All technical terms used in the present disclosure, unless otherwise defined, are used as the meaning as commonly understood by those skilled in the related art of the present disclosure. In addition, although preferred methods and samples are described herein, similar or equivalent methods and samples thereto are also included in the scope of the present disclosure. The contents of all publications disclosed herein by reference are incorporated in the present disclosure.

Throughout this specification, '%' used to indicate the concentration of a specific material is solid/solid (w/w)%, solid/liquid (w/v)%, and liquid/liquid (v/v)%, unless otherwise stated.

In one aspect, the present disclosure relates to a pharmaceutical composition for preventing or treating neuropathic pain, including a compound YJ102 represented by Chemical Formula 1, an isomer thereof, a solvate thereof, a hydrate thereof, or a salt thereof as an active ingredient:

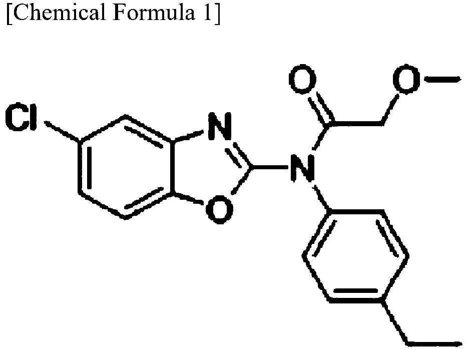

In one embodiment, the neuropathic pain may be neuropathic pain caused by nerve injury, and may be pain caused by peripheral or central nerve injury or dysfunction.

In one embodiment, the nerve injury may be multiple sclerosis, Parkinson's disease, stroke, nerve injury caused by diabetes, tumors or malformation of the spinal cord, spinal cord injury caused by trauma or inflammation, or spinal stenosis.

In one embodiment, the neuropathic pain may be diabetic peripheral neuropathic pain, central sensitization, shingles, postherpetic neuralgia, trigeminal neuralgia, complex regional pain syndrome, reflex sympathetic dystrophy, migraine, phantom limb pain, neuropathic pain caused by trauma, neuropathic pain caused by nerve injury, neuropathic pain caused by spinal stenosis, neuropathic pain caused by spinal cord injury, neuropathic pain caused by chronic diseases (multiple sclerosis, HIV, etc.), neuropathic pain caused by trauma (causalgia), neuropathic pain caused by impingement (i.e., sciatica, carpal tunnel, etc.), neuropathic pain caused by drug or toxic chemical exposure, neuropathic pain caused by infection or post-infection, neuropathic pain caused by damaged organ functions, neuropathic pain caused by vascular diseases, neuropathic pain caused by metabolic diseases, neuropathic pain caused by cancer or cancer treatment, neuropathic pain caused by autoimmune diseases, neuropathic back pain, neuropathic pain caused by fibromyalgia, or idiopathic neuropathic pain.

In one embodiment, the composition of the present disclosure may inhibit the activation of microglia or astrocytes.

In one embodiment, the composition of the present disclosure may inhibit the expression of Jumonji domain-containing protein 3 (JMJD3), transient receptor potential cation channel (TRPM7, subfamily M, member 7), IL-6, iNOS, COX-2, IL-1β, TNF-α, MIP-1α, MIP-1β, MIP-2α, Gro-α, or MCP-1.

In one embodiment, the composition of the present disclosure may inhibit phosphorylation of p38MAPK, JAK2, STAT3, S6 or p70S6 kinase.

In one embodiment, the composition of the present disclosure may increase the level of Trimethylated histone H3 at lysine 27 (H3K27Me3).

In one aspect, the present disclosure relates to a pharmaceutical composition for preventing or treating neuroinflammation, including a compound represented by Chemical Formula 1, an isomer thereof, a solvate thereof, a hydrate thereof, or a salt thereof as an active ingredient:

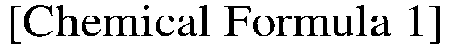

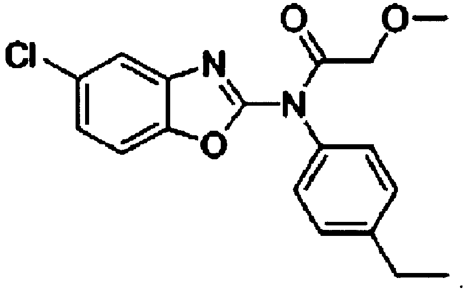

In one embodiment, the neuroinflammation may be caused by nerve injury, and the nerve injury may be spinal cord injury caused by trauma or inflammation or spinal stenosis.

In one embodiment, the neuroinflammation may be caused by the activation of microglia or astrocytes.

In one embodiment, the composition of the present disclosure may inhibit the expression of Jumonji domain-containing protein 3 (JMJD3), transient receptor potential cation channel (TRPM7, subfamily M, member 7), IL-6, iNOS, COX-2, IL-1β, TNF-α, MIP-1α, MIP-1β, MIP-2α, Gro-α, or MCP-1.

In one embodiment, the composition of the present disclosure may inhibit phosphorylation of p38MAPK, JAK2, STAT3, S6 or p70S6 kinase.

In one aspect, the present disclosure relates to a pharmaceutical composition for preventing or treating bladder diseases, including a compound YJ102 represented by Chemical Formula 1, an isomer thereof, a solvate thereof, a hydrate thereof, or a salt thereof as an active ingredient:

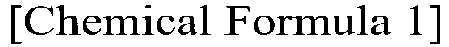

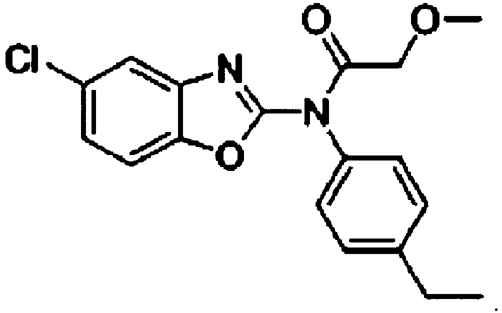

In one embodiment, the bladder diseases may be urolithiasis, cystitis, prostate cancer, urinary incontinence, hematuria, bladder cancer, urethritis, prostatitis, urinary frequency, enuresis, urgency, oliguria, acute cystitis, hypospadias, urodynia, bladder stones, urethrostenosis, overactive bladder syndrome, chronic cystitis, urinary tract infections, genuine stress urinary incontinence, chronic prostatitis, urinary hesitancy, or chronic bladder failure.

In one embodiment, the bladder diseases may be caused by spinal cord injury, and the spinal cord injury may be caused by trauma or inflammation.

In one embodiment, the spinal cord injury may be traumatic spinal cord injury, degenerative spinal diseases, inflammatory spinal diseases, spinal cord tumor, spinal cord malformation, spinal tumor, spinal cord hemorrhage, stroke, spinal cord paralysis caused by extramedullary vascular disorder, myelitis, multiple sclerosis, amyotrophic lateral sclerosis, or myelopathy.

In one embodiment, the composition of the present disclosure may inhibit bladder hypertrophy.

In one embodiment, the composition of the present disclosure may increase the expression of ZO-1 and occludin in the urothelium.

In one embodiment, the composition of the present disclosure may protect urothelial cells and inhibit apoptosis of urothelial cells.

In one embodiment, the composition of the present disclosure may inhibit the expression of p75, NGF, proNGF, COX-2, iNOS, IL-6, IL-1β, MIP-1α, MIP-1β, MIP-2α or Gro-α.

In one aspect, the present disclosure relates to a pharmaceutical composition for preventing or treating neurogenic bladder, including a compound YJ102 represented by Chemical Formula 1, an isomer thereof, a solvate thereof, a hydrate thereof, or a salt thereof as an active ingredient:

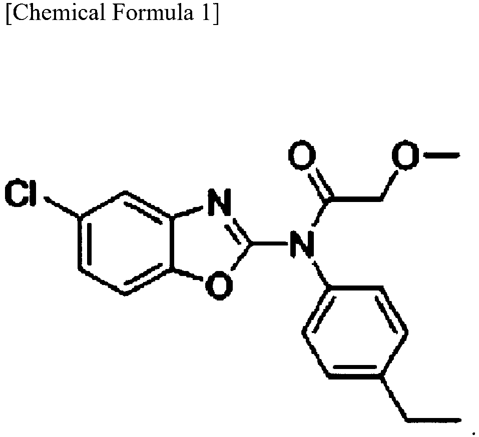

In one embodiment, the neurogenic bladder may be caused by spinal cord injury, and the spinal cord injury may be caused by trauma or inflammation.

In one embodiment, the spinal cord injury may be traumatic spinal cord injury, degenerative spinal disease, inflammatory spinal disease, spinal cord tumor, spinal cord malformation, spinal tumor, spinal cord hemorrhage, stroke, spinal cord paralysis caused by extramedullary vascular disorder, myelitis, multiple sclerosis, amyotrophic lateral sclerosis, or myelopathy.

In one embodiment, the neurogenic bladder may exhibit symptoms of urinary retention, urinary urgency, urgency incontinence, urinary frequency, nocturia, dysuria, urinary disturbances, or pyelonephritis.

In one embodiment, the composition of the present disclosure may inhibit bladder hypertrophy.

In one embodiment, the composition of the present disclosure may increase the expression of ZO-1 and occludin in the urothelium.

In one embodiment, the composition of the present disclosure may protect urothelial cells and inhibit apoptosis of the urothelial cells.

In one embodiment, the composition of the present disclosure may inhibit the expression of p75, NGF, proNGF, COX-2, iNOS, IL-6, IL-1β, MIP-1α, MIP-1β, MIP-2α or Gro-α.

In one embodiment, the composition of the present disclosure includes a pharmaceutically acceptable salt of the compound represented by Chemical Formula 1, and any possible solvate, hydrate, racemate or stereoisomer thereof that may be prepared therefrom.

In the present disclosure, the hydrate may be formed by binding the compound represented by Chemical Formula 1, a pharmaceutically acceptable salt thereof, or a stereoisomer thereof with water by a non-covalent intermolecular force, and may contain a stoichiometric or non-stoichiometric amount of water. Specifically, the hydrate may contain water in a ratio of about 0.25 mole to about 10 moles based on 1 mole of the active ingredient, and more specifically, about 0.5 mole, about 1 mole, about 1.5 moles, about 2 moles, about 2.5 moles, about 3 moles, about 5 moles, etc.

In the present disclosure, the solvate may be formed by binding the compound represented by Chemical Formula 1, a pharmaceutically acceptable salt thereof, or a stereoisomer thereof with a solvent other than water by an intermolecular force, and may contain a stoichiometric or non-stoichiometric amount of solvent. Specifically, the solvate may contain solvent molecules in a ratio of about 0.25 mole to about 10 moles based on 1 mole of the active ingredient, and more specifically, about 0.5 mole, about 1 mole, about 1.5 moles, about 2 moles, about 2.5 moles, about 3 moles, about 5 moles, etc.

The compound YJ102 represented by Chemical Formula 1 of the present disclosure may be used in the form of a pharmaceutically acceptable salt, and as the salt, an acid addition salt formed by a pharmaceutically acceptable free acid is useful. The acid addition salt is obtained from inorganic acids, such as hydrochloric acid, nitric acid, phosphoric acid, sulfuric acid, hydrobromic acid, hydroiodic acid, nitrous acid or phosphorous acid, and non-toxic organic acids, such as aliphatic mono- and di-carboxylates, phenyl-substituted alkanoates, hydroxy alkanoates and alkandioates, aromatic acids, and aliphatic and aromatic sulfonic acids. These pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, hydroxybutyrate, glycolate, malate, tartrate, methanesulfonate, propanesulfonate, naphthalene-1-sulfonate, naphthalene-2-sulfonate or mandelate.

The acid addition salts according to the present disclosure may be prepared by a conventional method, for example, by dissolving the compound YJ102 represented by Chemical Formula 1 in an excess acidic aqueous solution and precipitating the salt using a water-miscible organic solvent, such as methanol, ethanol, acetone or acetonitrile. In addition, the acid addition salts may also be prepared by evaporating and drying a solvent or excess acid from the mixture or by suction-filtering the precipitated salt.

In addition, pharmaceutically acceptable metal salts may be prepared using bases. Alkali metal or alkaline earth metal salts may be obtained, for example, by dissolving the compound in an excess alkali metal hydroxide or alkaline earth metal hydroxide solution, filtering a non-dissolved compound salt, and then evaporating and drying a filtrate. At this time, as the metal salt, it is pharmaceutically suitable to prepare a sodium, potassium or calcium salt. Further, salts corresponding thereto may be obtained by reacting the alkali metal or alkaline earth metal salt with a suitable silver salt (e.g., silver nitrate).

The pharmaceutical composition of the present disclosure may further include a known therapeutic agent in addition to the compound represented by Chemical Formula 1 as an active ingredient, and may be used in combination with other known treatments for the treatment of the disease.

In the present disclosure, the term "prevention" means all actions of inhibiting or delaying the occurrence, spread, and recurrence of the disease by administering the pharmaceutical composition according to the present disclosure, and "treatment" means all actions of improving or beneficially changing the symptoms of the disease by administering the composition of the present disclosure. Those skilled in the art to which the present disclosure pertains will be able to determine the degree of alleviation, enhancement and treatment by knowing the exact criteria of disease for which the composition of the present disclosure is effective by referring to data presented by the Korean Academy of Medical Sciences, etc.

In the present disclosure, the term "therapeutically effective amount" used in combination with the active ingredient means an amount effective to prevent or treat the disease, and the therapeutically effective amount of the composition of the present disclosure may vary depending on several factors, such as a method of administration, a target site, the condition of a patient. Accordingly, when used in the human body, a dose should be determined as an appropriate amount in consideration of both safety and efficiency. It is also possible to estimate an amount to be used in humans from the effective amount determined through animal experiments. These matters to be considered when determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present disclosure is administered in a pharmaceutically effective amount. In the present disclosure, the term "pharmaceutically effective amount" refers to an amount enough to treat the disease at a reasonable benefit/risk ratio applicable to medical treatment and enough not to cause side effects. The effective dose level may be determined according to factors including the health condition of a patient, the type and severity of a disease, the activity of a drug, the sensitivity to a drug, a method of administration, a time of administration, a route of administration, an excretion rate, duration of treatment, and drugs used in combination or simultaneously, and other factors well-known in the medical field. The composition of the present disclosure may be administered as a separate therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present disclosure may include a carrier, a diluent, an excipient, or a combination of two or more thereof, which are commonly used in biological agents. In the present disclosure, the term "pharmaceutically acceptable" refers to exhibiting non-toxic properties to cells or humans exposed to the composition. The carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

In one embodiment, the pharmaceutical composition may be one or more formulations selected from the group including oral formulations, external formulations, suppositories, sterile injection solutions and sprays.

In the present disclosure, the term "administration" means providing a predetermined substance to a subject or patient by any suitable method, and the pharmaceutical composition may be administered parenterally (e.g., applied with injectable formulations intravenously, subcutaneously, intraperitoneally or topically) or orally according to a desired method. The dose range may vary depending on the body weight, age, sex, and health condition of a patient, a diet, an administration time, an administration method, an excretion rate, the severity of pain, and the like. Liquid formulations for oral administration of the composition of the present disclosure correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like. The pharmaceutical composition of the present disclosure may also be administered by any device capable of moving an active substance to a target site. Preferred administration methods and formulations are intravenous injections, subcutaneous injections, intradermal injections, intramuscular injections, drop injections, etc. The injections may be prepared by using aqueous solvents such as a physiological saline solution and a ringer solution, and non-aqueous solvents such as vegetable oils, higher fatty acid esters (e.g., ethyl oleate), and alcohols (e.g., ethanol, benzyl alcohol, propylene glycol, or glycerin). The injections may include pharmaceutical carriers, such as a stabilizer (e.g., ascorbic acid, sodium hydrogen sulfite, sodium pyrosulfite, BHA, tocopherol, EDTA, etc.) for the prevention of degeneration, an emulsifier, a buffer for pH control, and a preservative (e.g., phenyl mercury nitrate, thimerosal, benzalkonium chloride, phenol, cresol, benzyl alcohol, etc.) to inhibit microbial growth.

In the present disclosure, the term "subject" refers to all animals including monkeys, cows, horses, sheep, pigs, chickens, turkeys, quails, cats, dogs, mice, rats, rabbits or guinea pigs including humans who have developed or may develop the disease, and the pharmaceutical composition of the present disclosure may be administered to a subject to effectively prevent or treat the disease. The pharmaceutical composition of the present disclosure may be administered in combination with existing therapeutic agents.

The pharmaceutical composition of the present disclosure may further include a pharmaceutically acceptable additive. At this time, the pharmaceutically acceptable additive may be used with starch, gelatinized starch, microcrystalline cellulose, lactose, povidone, colloidal silicon dioxide, calcium hydrogen phosphate, lactose, mannitol, syrup, Arabic gum, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc and the like. The pharmaceutically acceptable additive according to the present disclosure is preferably included in an amount of 0.1 part by weight to 90 parts by weight based on the composition, but is not limited thereto.

In the present disclosure, the term "expression" generally refers to a cellular process in which a biologically active polypeptide is produced from a DNA sequence and exhibits biological activity in a cell. In that meaning, the gene expression includes not only transcription and translation processes, but also post-transcriptional and post-translational processes that may affect the biological activity of a gene or gene product. The processes include not only RNA synthesis, processing and transport, but also polypeptide synthesis, transport and post-translational modifications, but are not limited thereto.

In the present disclosure, the expression may be confirmed by measuring the expression level of a gene or mRNA by polymerase chain reaction (PCR), real-time RT-PCR, reverse transcription PCR, competitive RT-PCR, nuclease protection analysis (RNase, S1 nuclease assay), in situ hybridization, nucleic acid microarray, Northern blot, or DNA chip method using a primer pair, a probe, or both a primer pair and a probe that specifically recognizes a nucleic acid sequence, a nucleic acid sequence complementary to the nucleic acid sequence, and fragments of the nucleic acid sequence and the complementary sequence. In addition, the expression may be confirmed by measuring the expression level of a protein by Western blot, enzyme linked immunosorbent assay (ELISA), radioimmunoassay (RIA), radioimmunodiffusion, immunoelectrophoresis, immunohistochemical staining, immunoprecipitation assay, complement fixation assay, FACS, mass spectrometry, or protein microarray using antibodies, antibody fragments, aptamers, avidity multimers, or peptidomimetics that specifically recognize a full-length protein or fragments thereof.

In one aspect, the present disclosure relates to a composition for alleviating bladder dysfunction including a compound represented by Chemical Formula 1 of the present disclosure, an isomer thereof, a solvate thereof, a hydrate thereof, or a salt thereof as an active ingredient:

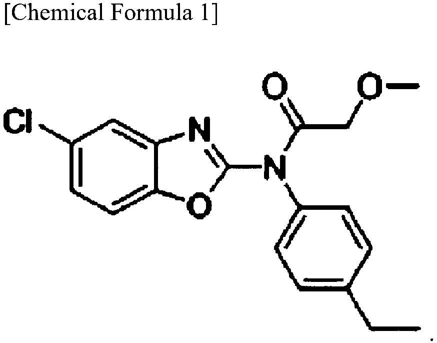

In one embodiment, the bladder dysfunction may be bladder dysfunction caused by spinal cord injury. The spinal cord injury may be traumatic spinal cord injury, spinal degenerative diseases, spinal inflammatory diseases, spinal cord tumor, spinal cord malformation, spinal tumor, spinal cord hemorrhage, stroke, spinal cord paralysis caused by extramedullary vascular disorder, myelitis, multiple sclerosis, amyotrophic lateral sclerosis, or myelopathy.

In one embodiment, the composition of the present disclosure may alleviate or restore the urinary functions of the bladder.

In one aspect, the present disclosure relates to a composition for inhibiting bladder hypertrophy including a compound represented by Chemical Formula 1 of the present disclosure, an isomer thereof, a solvate thereof, a hydrate thereof, or a salt thereof as an active ingredient:

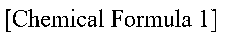

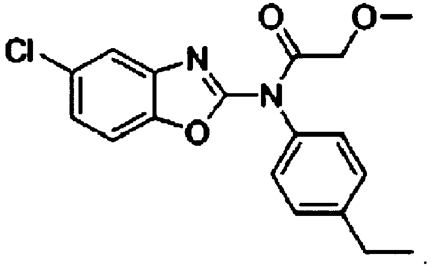

In one embodiment, the composition may inhibit an increase in the thickness of the bladder wall, i.e., the urothelial layer, the lamina propria, or the muscular layer of the bladder.

In one aspect, the present disclosure relates to a food composition for preventing or alleviating neuropathic pain, including a compound represented by Chemical Formula 1 of the present disclosure, an isomer thereof, a solvate thereof, a hydrate thereof, or a salt thereof:

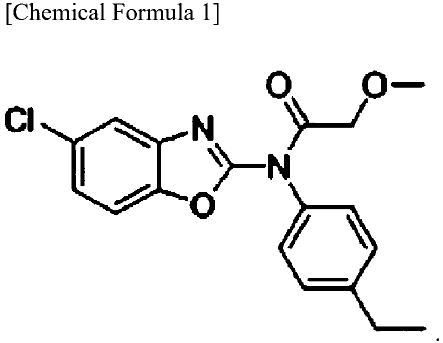

In one aspect, the present disclosure relates to a food composition for preventing or alleviating neuroinflammation, including a compound represented by Chemical Formula 1 of the present disclosure, an isomer thereof, a solvate thereof, a hydrate thereof, or a salt thereof:

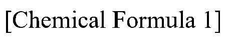

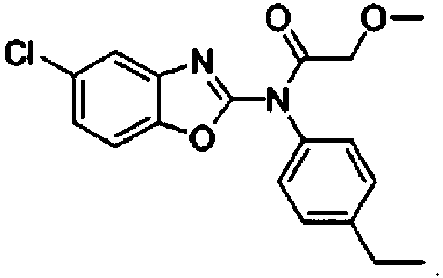

In one aspect, the present disclosure relates to a food composition for preventing or alleviating bladder diseases, including a compound represented by Chemical Formula 1 of the present disclosure, an isomer thereof, a solvate thereof, a hydrate thereof, or a salt thereof:

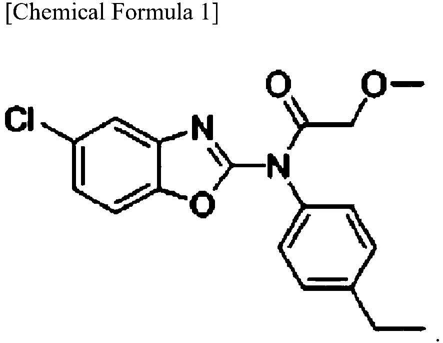

In one aspect, the present disclosure relates to a food composition for preventing or alleviating neurogenic bladder, including a compound represented by Chemical Formula 1 of the present disclosure, an isomer thereof, a solvate thereof, a hydrate thereof, or a salt thereof:

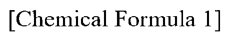

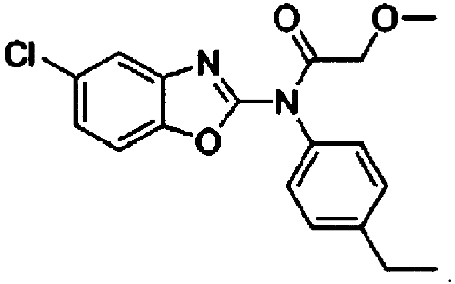

In one aspect, the present disclosure relates to a food composition for alleviating bladder functions or inhibiting bladder hypertrophy, including a compound represented by Chemical Formula 1 of the present disclosure, an isomer thereof, a solvate thereof, a hydrate thereof, or a salt thereof:

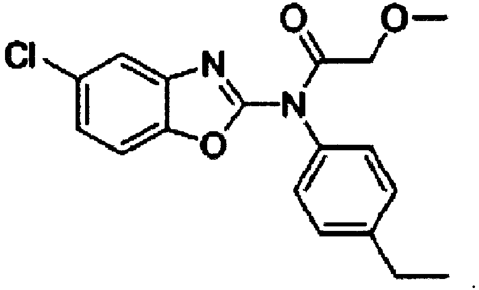

When the composition of the present disclosure is used as the food composition, the compound represented by Chemical Formula 1, an isomer thereof, a solvate thereof, a hydrate thereof, or a salt thereof may be added as it is or used together with other foods or food ingredients, and may be used appropriately according to a conventional method. The composition may include food acceptable supplement additives in addition to the active ingredients, and a mixing amount of the active ingredients may be appropriately determined depending on the purpose of use (prevention, health or therapeutic treatment).

As used in the present disclosure, the term "food supplement additive" means a component that may be supplementarily added to food, and may be appropriately selected and used by those skilled in the art as being added to prepare a health functional food of each formulation. Examples of the food supplement additive include various nutrients, vitamins, minerals (electrolytes), flavors such as synthetic and natural flavors, colorants and fillers, pectic acid and salts thereof, alginic acid and salts thereof, organic acids, protective colloidal thickeners, pH adjusters, stabilizers, preservatives, glycerin, alcohols, carbonation agents used in carbonated drinks, and the like, but the types of food supplement additive of the present disclosure are not limited by the examples.

The food composition of the present disclosure may include a health functional food. As used in the present disclosure, the term "health functional food" refers to food prepared and processed in the form of tablets, capsules, powders, granules, liquids and pills by using raw materials or ingredients having functionalities useful to the human body. Here, the "functionality" means adjusting nutrients or obtaining effects useful for health applications such as physiological action with respect to the structures and functions of the human body. The health functional food of the present disclosure is able to be prepared by methods to be commonly used in the art and may be prepared by adding raw materials and ingredients which are commonly added in the art in preparation. In addition, the formulations of the health functional food may also be prepared with any formulation recognized as a health functional food without limitation. The food composition of the present disclosure may be prepared in various forms of formulations, and the health functional food of the present disclosure may be consumed as a supplement to enhance the effect of an analgesic.

In addition, there is no limitation in types of health food in which the composition of the present disclosure may be used. In addition, the composition including the compound represented by Chemical Formula 1 of the present disclosure, an isomer thereof, a solvate thereof, a hydrate thereof, or a pharmaceutically acceptable salt thereof as an active ingredient may be prepared by mixing other appropriate supplement ingredients that may be contained in the health functional food and known additives according to the selection of those skilled in the art. Examples of foods to be added include meat, sausage, bread, chocolate, candy, snacks, confectionery, pizza, ramen, other noodles, gum, dairy products including ice cream, various soups, beverages, tea, drinks, alcoholic beverages, vitamin complexes and the like, and may be prepared to be added to extract, tea, jelly, juice, and the like prepared by using the compound according to the present disclosure as a main ingredient.

In one aspect, the present disclosure relates to a use of a compound represented by Chemical Formula 1 of the present disclosure, an isomer thereof, a solvate thereof, a hydrate thereof, or a salt thereof, for the preparation of a pharmaceutical composition for preventing or treating neuropathic pain, neuroinflammation, bladder diseases, neurogenic bladder or inflammatory skin diseases.

In one aspect, the present disclosure relates to a use of a compound represented by Chemical Formula 1 of the present disclosure, an isomer thereof, a solvate thereof, a hydrate thereof, or a salt thereof, for the preparation of a composition for alleviating bladder dysfunction or inhibiting bladder hypertrophy.

In one aspect, the present disclosure relates to a method for treating neuropathic pain, neuroinflammation, bladder diseases, neurogenic bladder or inflammatory skin diseases, including administering a compound represented by Chemical Formula 1 of the present disclosure, an isomer thereof, a solvate thereof, a hydrate thereof, or a salt thereof to a subject suffering from neuropathic pain, neuroinflammation, bladder diseases, neurogenic bladder or inflammatory skin diseases.

In one aspect, the present disclosure relates to a method for treating or alleviating bladder dysfunction or bladder hypertrophy, including administering a compound represented by Chemical Formula 1 of the present disclosure, an isomer thereof, a solvate thereof, a hydrate thereof, or a salt thereof to a subject having symptoms of bladder dysfunction or bladder hypertrophy.

### [Modes]

Hereinafter, the present disclosure will be described in more detail through the following Examples. However, the following Examples are only intended to embody the contents of the present disclosure, and the present disclosure is not limited thereto.

### Example 1. Preparation of nerve injury animal model

### 1-1. Preparation of central nerve injury animal model

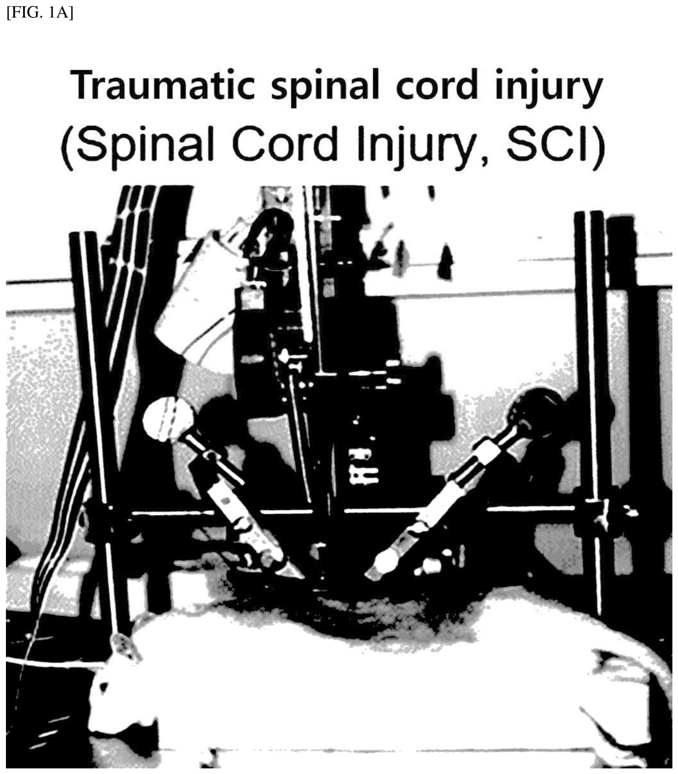

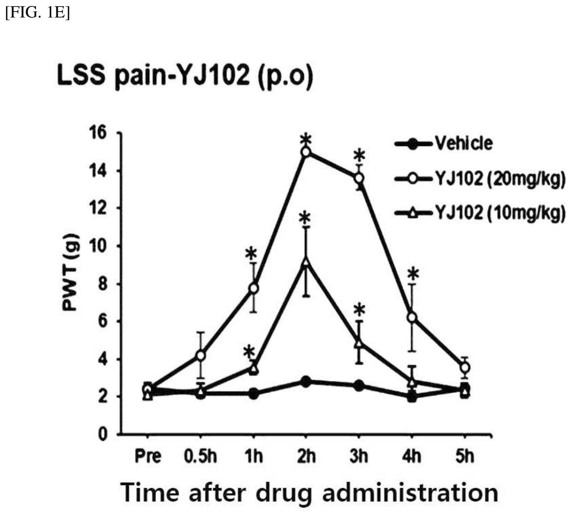

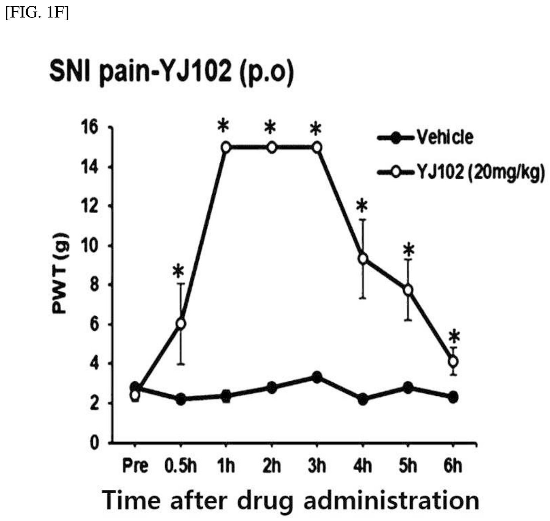

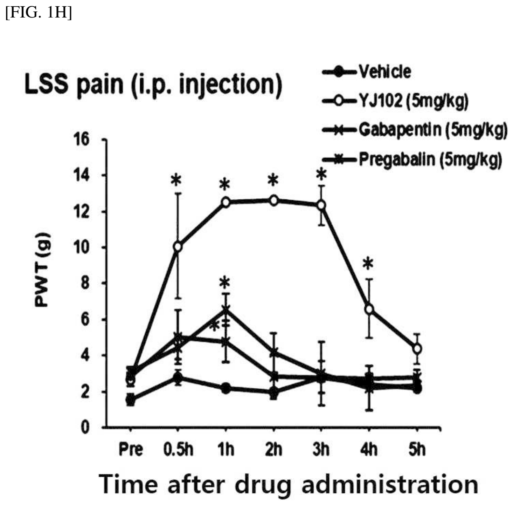

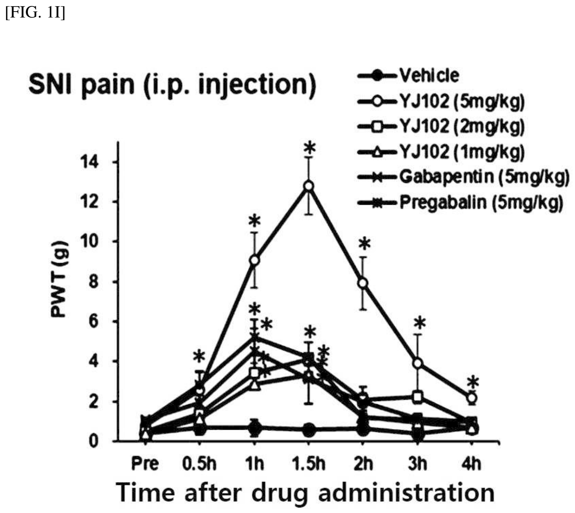

To be used as a central nerve injury animal model, a traumatic spinal cord injury (SCI) model was prepared (FIG. 1A). Specifically, 230 to 250 g of male adult Sprague-Dawley rats were anesthetized by intraperitoneal injection of chloral hydrate (500 mg/kg), and then the back and neck regions were shaved, and the spinal cord under the back and neck regions was exposed without damaging the dura mater, and a laminectomy was performed at T9 to T10 levels. To stabilize the spine, the spinous processes of T8 and T11 were fixed, and the spine was damaged by free-falling a 10 g weight from a height of 25 mm using an NYU impactor onto the back surface of the exposed spinal cord and then the rats were left in a temperature and humidity-controlled chamber overnight while covering the muscles and skin. After surgery, the rats were subcutaneously injected with a supplement solution (5 ml, lactated ringer) and injected with antibiotics (gentamicin, 5 mg/kg, intramuscular injection) once daily for 5 days. The body weight, remaining food, and the weight of water were confirmed and recorded for all animals, and the bladder was emptied twice daily by passive bladder massage until reflex micturition was established. At this time, a Sham group was a group that was subjected to only laminectomy without damage.

### 1-2. Preparation of peripheral nerve injury animal model

### 1-2-1. Lumbar spinal stenosis model

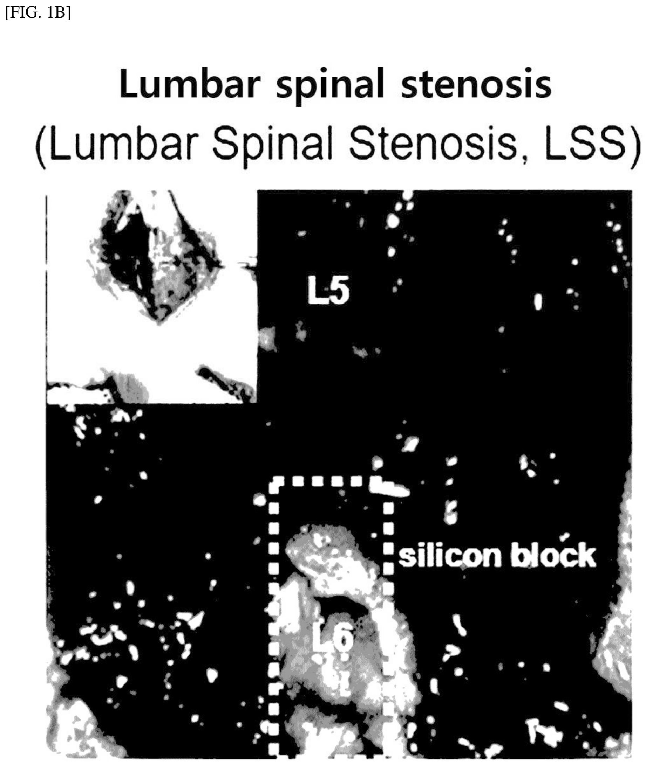

To be used as a peripheral nerve injury animal model, a lumbar spinal stenosis (LSS) model was prepared (FIG. 1B). Specifically, 260 to 280 g of adult male Sprague-Dawley rats were anesthetized with intraperitoneal injection of chloral hydrate (500 mg/kg), the back region was shaved, and then the spinal plate was exposed at L4 to S2 levels. After removing the ligamentous section between L4 and L5, a trapezoidal silicone block (1.00 mm long × 1.3 to 1.2 mm wide × 1.0 mm high) was inserted into the epidural space below the L5 and L6 spinal plates without damaging the dural sac. At this time, a Sham group was a group that was subjected to only posterior laparotomy on the animal without inserting a silicone block.

### 1-2-2. Sciatic nerve injury (SNI) model

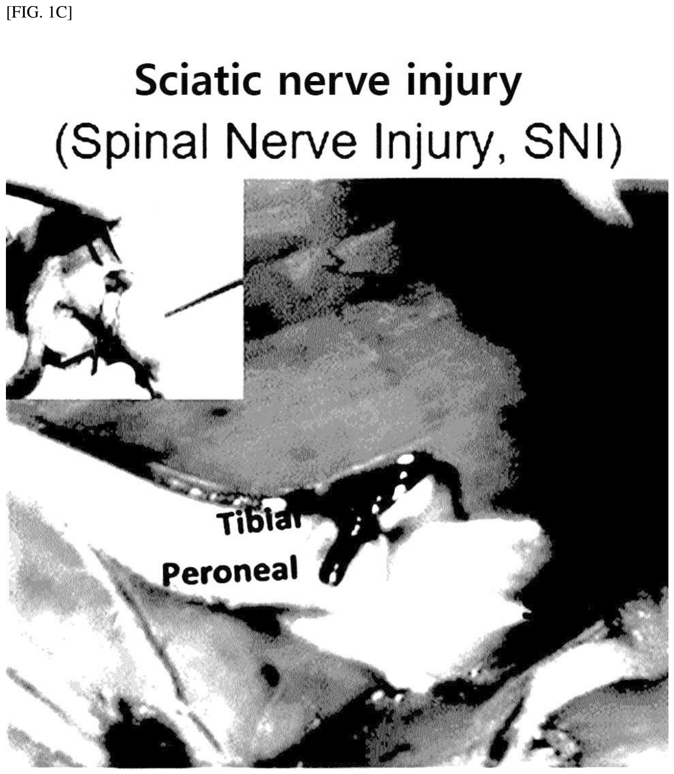

To be used as a peripheral nerve injury animal model, a sciatic nerve injury (SNI) model was prepared (FIG. 1C). Specifically, 200 to 220 g of male adult Sprague-Dawley rats were anesthetized by intraperitoneal injection of chloral hydrate (500 mg/kg), and then three terminal branches (tibial, common peroneal, and sural nerves) of the right sciatic nerve were approached, and the tibia nerve and the peroneal nerve were tightly ligated with 4-0 silk, and neurectomy was performed at a distal 3 mm position of the ligation site. At this time, a Sham group was a group that exposed only the sciatic nerve without damage.

### Example 2. Confirmation of neuropathic pain inhibitory effect of compound of the present disclosure

In order to confirm a neuropathic pain inhibitory effect of YJ102 (Chemical Formula 1), a compound of the present disclosure, in the three nerve injury animal models prepared in Example 1 above, rats having pain on week 3 (SNI model) or week 4 (SCI model and LSS model) after surgery were selected and administered orally (p.o.) at 10 mg/kg or 20 mg/kg of YJ102 dissolved in a solution of DMSO, methyl pyrrolidinone, and water (1 : 1.5 : 1), or intraperitoneally injected (i.p. injection) at 1, 2, or 5 mg/kg of YJ102. In a positive control group, gabapentin or pregabalin dissolved in saline was intraperitoneally injected at a concentration of 5 mg/kg, and in a Vehicle group, the same amount of a solution of DMSO, methyl pyrrolidinone, and water was administered. Thereafter, in order to measure mechanical allodynia, the rats were located at the bottom of a transparent plastic box (10 cm × 10 cm) on top of a wire mesh (3 mm × 3 mm) and subjected to an adaptation period for approximately 20 minutes. Von-Frey filaments were applied to the soles of both paws and used with stiff nylon von-Frey monofilament series (3.61, 3.84, 4.08, 4.31, 4.56, 4.74, 4.93 and 5.18 mN, Stoelting, WoodDale, IL, USA; equivalent in grams to 0.4, 0.6, 1.0, 2.0, 4.0, 6.0, 8.0 and 15.0) with logarithmic increases in tactile (mechanical) sensitivity. The avoidance response of the paws to increased mechanical stimulation was evaluated by applying a sufficient force. The von-Frey filaments were applied to each paw for 3 to 4 seconds to be fully bent, and a 50% threshold was determined using an up-down method (Chaplan et al. 1994). 2.0 g (4.31 mN) of filaments, which were the middle of 0.4 to 15 g of eight von-Frey filaments, were first used and the most sensitive area was identified at various locations. When no avoidance response of the paws was observed, the experiment was repeated with the next thicker filaments. When the avoidance response occurred, weaker filaments were applied to the sensitive area. Stimulations were performed at 2-second intervals. The 50% threshold was expressed as gram (g) of von Frey hair that showed a 50% avoidance response to sole stimulation, and measured at both paws to calculate the average value, and a statistically significant decrease in the avoidance threshold compared to before spinal cord injury induction was determined to be the occurrence of mechanical allodynia.

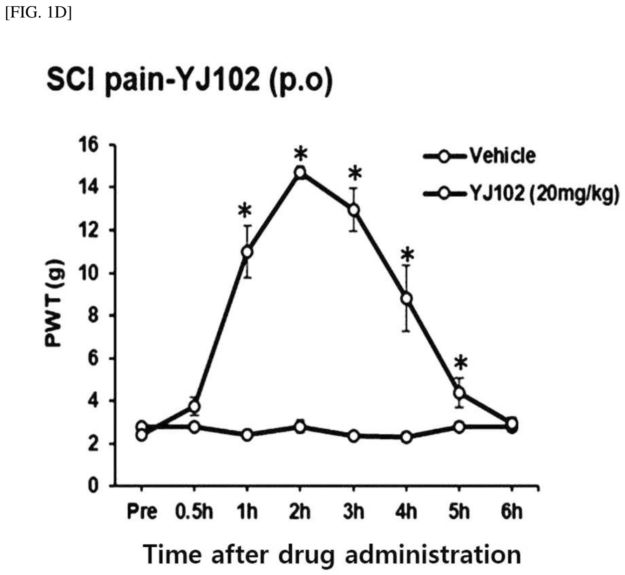

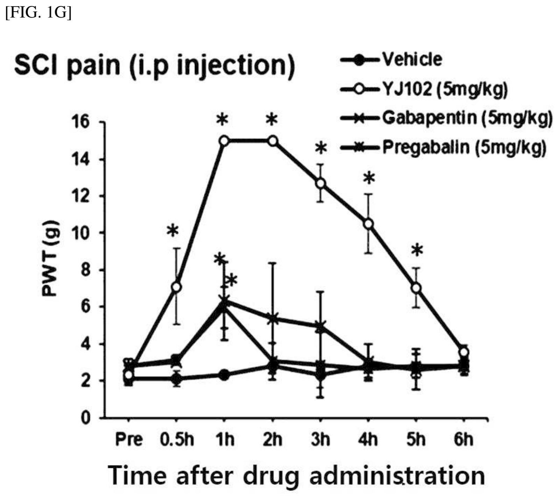

As a result, when mechanical allodynia was measured using von-Frey filaments, in rats 4 weeks after spinal cord injury, 4 weeks after induction of lumbar spinal stenosis, and 3 weeks after nerve injury, paw withdrawal threshold (PWT) scores were significantly lower than those in normal rats (15 points), showing 2 to 3 points or less (FIGS. 1D to 1F). Thus, it was confirmed that all three animal models had lower pain thresholds and induced neuropathic pain in which pain was felt even by stimuli that were not painful at all under normal conditions. In contrast, in all of the three animal models induced with neuropathic pain, when YJ102 was orally administered at 10 mg/kg or 20 mg/kg, the mechanical allodynia threshold was increased from 30 minutes after administration, and the analgesic effect lasted for 4 hours or more (FIGS. 1D to 1F). In addition, when YJ102 was administered intraperitoneally to compare the effects of gabapentin and pregabalin, which were currently prescribed for neuropathic pain in clinical practice, YJ102 showed an analgesic effect that was more than twice as high at the same concentration as gabapentin or pregabalin, which showed a significant analgesic effect at 1 hour after administration, and the duration of the analgesic effect was also found to be more than three times longer than that of existing drugs (FIGS. 1G to 1I). In particular, sedation as a side effect of the drug was observed when high concentrations of gabapentin (50 mg/kg) and pregabalin (20 mg/kg) were treated, whereas the sedation was not observed in a group treated with YJ102 even at a concentration of 100 mg/kg.

Through this, it was confirmed that the compound YJ102 of the present disclosure had a remarkable analgesic effect on neuropathic pain compared to drugs (gabapentene and pregabalin) used in conventional clinical practice, and particularly, it was confirmed that there were no side effects observed in conventional drugs even at high concentrations.

### Example 3. Analysis of mechanism of inhibiting neuropathic pain

### 3-1. Inhibition of microglial and astrocytic activation

To analyze the activation of microglia in the spinal cord, which were known to play an important role in the induction/persistence of neuropathic pain, 4 weeks after nerve injury in the SNI animal model, the spinal cord of 4 and 5 lumbar vertebrae was stained with OX-42 to confirm changes in cell shape and intensity. Specifically, an SNI animal model rat was perfused through the right atrium, and fixed with 4% paraformaldehyde, and the spinal cord nerves L4-L5 were cut, embedded in an optimum cutting temperature (OCT) compound, and the spinal cord tissue was sectioned at 10 µm thickness using a cryostat. Through immunohistochemical staining, each tissue section was treated with an anti-OX-42 antibody against OX-42 protein which wasa microglia marker, as a primary antibody, and reacted according to the manufacturer's protocol, and then color-developed with a secondary antibody (Jackson, USA). In addition, for protein isolation, the cut spinal cord tissue was perfused with PBS, cut into 1 cm sections on the damaged area, and homogenized by adding a Lysis Buffer [50 mM Tris-HCl (pH 7.4), 150 mM NaCl, 2 mM MgCl₂, 0.1% NP40]. The homogenized tissue sections were transferred to a microcentrifuge tube, rock at 4°C for 20 minutes, and then centrifuged at 25,000 × g for 10 minutes. The supernatant was transferred to a new microcentrifuge, the protein concentration was measured using a BCA kit, and samples for Western blot were prepared by adding a 1X sample buffer and boiling for 5 minutes. For Western blot, the same amount of protein was loaded onto SDS-PAGE, electrophored, and then transferred to a nitrocellulose membrane, and blotted using primary and secondary antibodies against p38MAPK and p-p38MAPK.

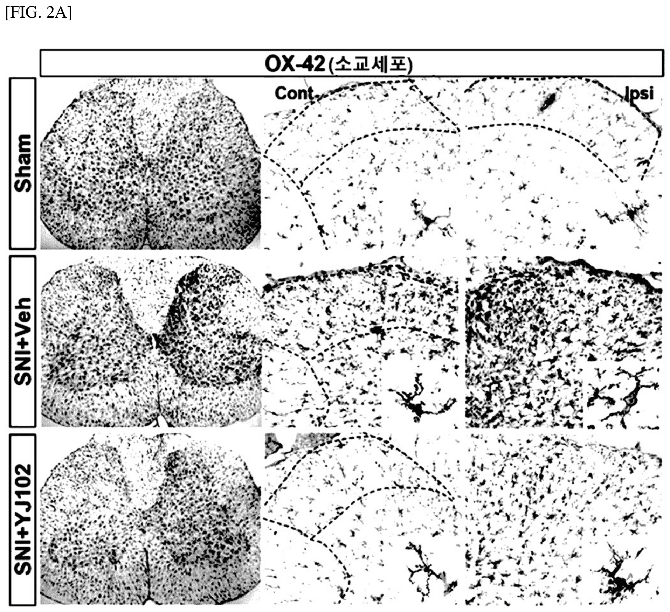

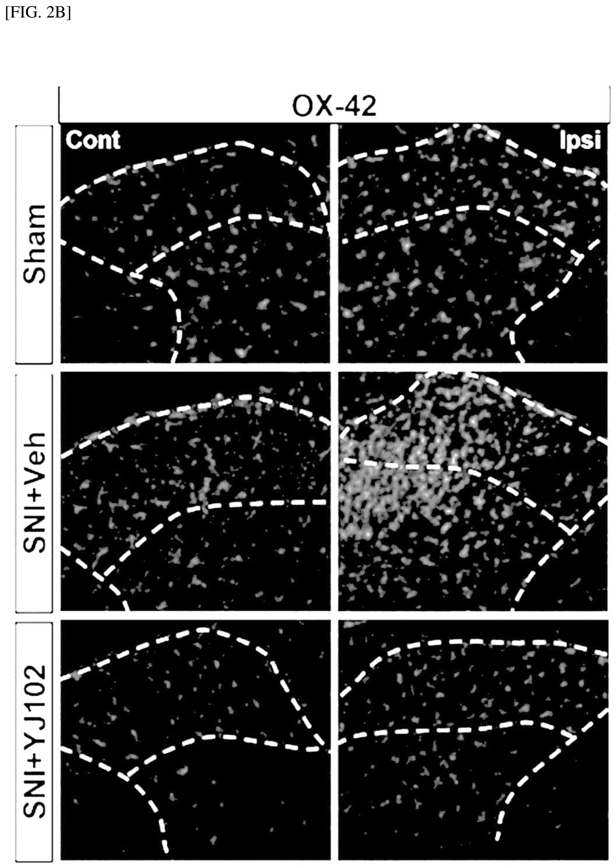

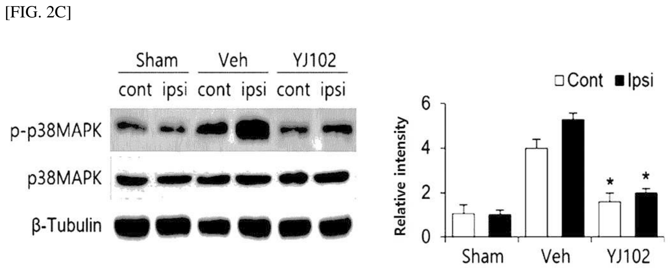

As a result, the microglia stained with OX-42 showed a significant increase in OX-42 intensity in a Vehicle administered group compared to the Sham group in both the dorsal horn (contralateral) tissue of a control group on a non-operated side and the ipsilateral tissue of the experimental group on a nerve-injured side, and typical activated microglia with thick and short branches were identified (FIGS. 2A and 2B). In contrast, it was shown that these activation changes were significantly reduced in rats administered with YJ102. In addition, even as a result of the phosphorylation analysis of p38MAPK, which was known to be observed only in activated microglia, it was shown that the amount of p-p38MAPK was significantly reduced in the rats administered with YJ102 (FIG. 2C). Through this, it was confirmed that YJ102 inhibited the activation of microglia in the spinal cord tissue of a neuropathic pain model.

### 3-2. Inhibition of astrocyte activation

The activation of astrocytes in the spinal cord, known to play an important role in the process of inducing/persisting neuropathic pain, was confirmed by immunohistochemical staining and Western blot analysis using an anti-GFAP antibody against an intermediate filament protein, Glial fibrillary acidic protein (GFAP) of astrocytes, a marker of astrocyte activation, as in Example 3-1.

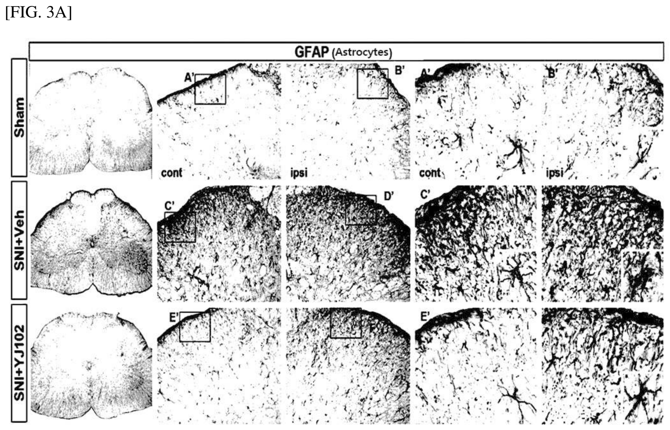

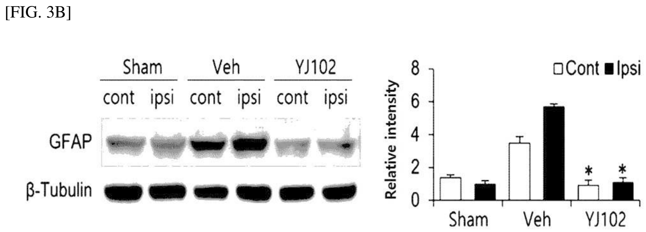

As a result, the expression of GFAP was significantly increased in the ipsilateral spinal cord compared to the contralateral spinal cord dorsal horn in the L4-5 Spinal cord tissue, and the astrocyte activation, which resulted in astrocyte hypertrophy, was observed (FIG. 3A). In contrast, in the group administered with YJ102, it was shown that the GFAP expression intensity was significantly decreased, which was equally shown even in the Western blot analysis results (FIGS. 3A and 3B). Through this, it was confirmed that YJ102 inhibited the activation of astrocytes in the spinal cord tissue of the neuropathic pain model.

### 3-3. Inhibition of inflammatory factor expression

It was known that when neuropathic pain occurred, the activation of microglia and astrocytes expressed inflammatory factors including cytokines and chemokines to cause excessive excitation of surrounding neurons. Therefore, in order to confirm changes in inflammatory factor expression by YJ102 administration in the SNI animal model 4 weeks after nerve injury, RNA and protein were extracted from L4-5 spinal cord tissue and RT-PCR and Western blot analysis were performed. Specifically, for RT-PCR analysis, RNA was isolated from spinal cord tissue sections using a TRIZOL Reagent (Invitrogen) according to a manual, and 20 µl of the total mixture was prepared by using 1 µg of total RNA, and cDNA was synthesized using MMLV reverse transcriptase (Invitrogen). IL-6, iNOS, COX-2, IL-1β, TNF-α, MIP-1α, MIP-1β, MIP-2α, Gro-α, and MCP-1 genes were amplified by using a cDNA template and primers in Table 1 below, respectively, and then electrophored on an agarose gel to obtain the results.

**[Table 1]**

| **Target** | **Primer** | **Sequence** |
|---|---|---|
| IL-1β | Forward | 5'-GCAGCTACCTATGTCTTGCCCGTG-3' |
| | Reverse | 5'-GTCGTTGCTTGTCTCTCCTTGTA-3' |
| TNF-α | Forward | 5'-CCCAGACCCTCACACTCAGAT-3' |
| | Reverse | S'-TTGTCCCTTGAAGAGAACCTG-3' |
| COX-2 | Forward | 5'-CCATGTCAAAACCGTGGTGAATG-3' |
| | Reverse | 5'-ATGGGAGTTGGGCAGTCATCAG-3' |
| iNOS | Forward | 5'-CTCCATGACTCTCAGCACAGAG-3 |
| | Reverse | 5'-GCACCGAAGATATCCTCATGAT-3' |
| MCP-1 | Forward | 5'-TCAGCCAGATGCAGTTAACG-3' |
| | Reverse | 5'-GATCCTCTTGTAGCTCTCCAGC-3' |
| MIP-1α | Forward | 5'-ACTGCCTGCTGCTTCTCCTACA-3' |
| | Reverse | 5'-AGGAAAATGACACCTGGCTGG-3 |
| MIP-1β | Forward | 5'-TCCCACTTCCTGCTGTTTCTCT-3' |
| | Reverse | 5'-GAATACCACAGCTGGCTTGGA-3 |
| Gro-α | Forward | 5'-CCGAAGTCATAGCCACACTCAA-3' |
| | Reverse | 5'-GCAGTCGTCTCTTTCTCCGTTAC-3' |
| MIP-2α | Forward | 5'-AGACAGAAGTCATAGCCACTCTCAAG-3' |
| | Reverse | 5 -CCTCCTTTCCAGGTCAGTTAGC-3' |
| GAPDH | Forward | 5'-AACTTTGGCATTGTGGAAGG-3' |
| | Reverse | S'-GGAGACAACCAGGTCCTCAG |

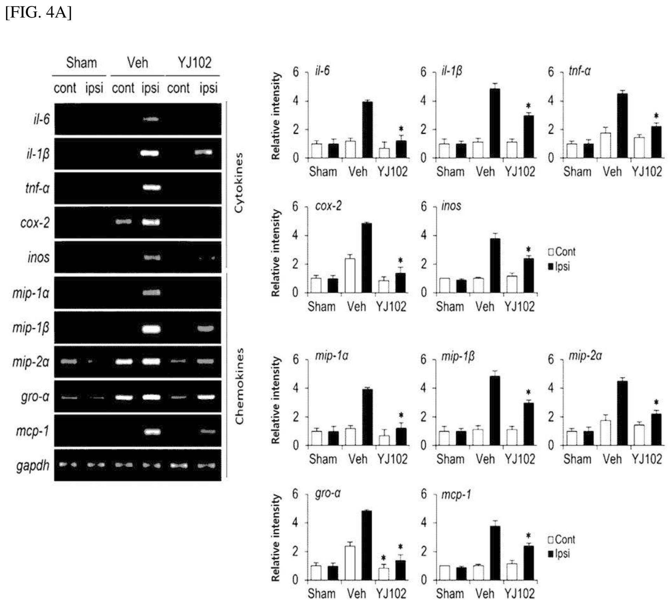

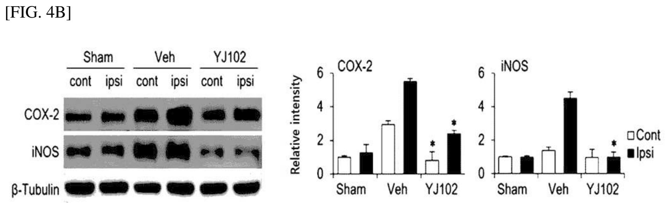

As a result, both RT-PCR and Western blot analyses showed that the inflammatory factors whose expression was markedly increased in the ipsilateral spinal cord tissue after SNI were significantly reduced in the group treated with YJ102 (FIG. 4).

### 3-4. Analysis of signaling pathways

To determine through which signaling pathway YJ102 inhibited a neuroinflammatory response, there was analyzed increased expression and activation of histone demethylase JMJD3 and expression changes of factors associated with JAK/STAT3 pathway, mTOR pathway, and TRPM7-mediated pathway, which were known as important regulatory mechanisms and epigenetic regulators mediating the neuroinflammatory response, in the tissues of the SNI animal model 4 weeks after nerve injury.

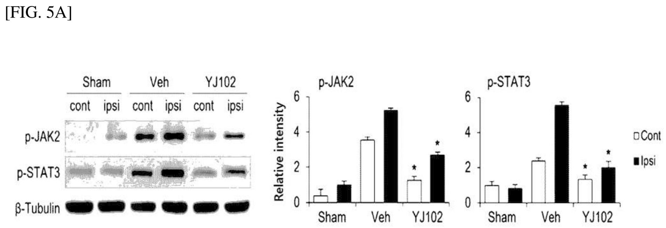

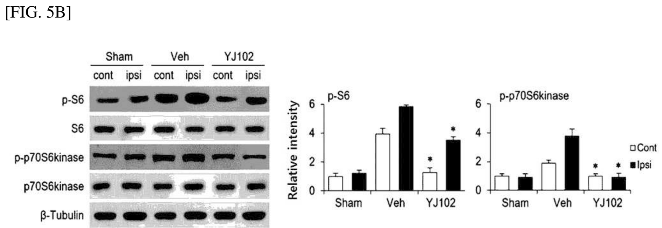

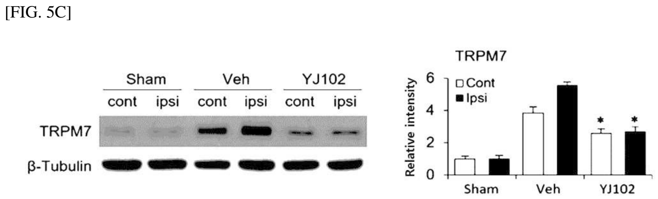

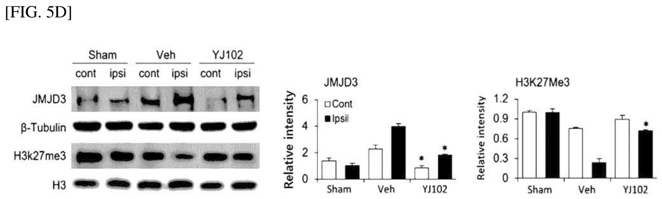

As a result, YJ102 inhibited the JAK/STAT3 pathway by significantly reducing the phosphorylation of JAK2 and STAT3, and also regulated the mTOR pathway by inhibiting the activity of S6 and p70S6kinase (FIG. 5). In addition, it was shown that YJ102 inhibited the expression of TRPM7, a non-selective cation ion channel that has been reported to cause neuropathic pain by mediating astrocyte activation (FIG. 5), but it was confirmed that YJ102 was associated with the inhibition of astrocyte activation by inhibiting the TRPM7 expression. In addition, it was shown that YJ102 significantly inhibited the expression of JMJD3 and the demethylation activation of H3K27Me3, which were factors involved in the expression of inflammatory factors such as IL-6 through histone demethylation, (FIG. 5), and as a result, there was a possibility to exhibit an anti-inflammatory effect.

### Example 4. Confirmation of effect of compound of the present disclosure on improving bladder functions

### 4-1. Urinary function analysis

In order to confirm the effect of YJ102 (Chemical Formula 1), a compound of the present disclosure, on urinary disturbances caused by spinal cord injury, immediately after preparing the traumatic spinal cord injury (SCI) model (FIG. 1A) prepared in Example 1 above, YJ102 was dissolved in DMSO, methyl pyrrolidinone, and water (1 : 1.5 : 1) and administered intraperitoneally at 5 mg/kg, and then intraperitoneally administered at 24-hour intervals for 7 days from the next day. Thereafter, the effect of improving bladder functions was analyzed through a residual urine volume test and a urinary bladder function score (UBFS). At this time, a Vehicle group was administered with the same amount of a solution of DMSO, methyl pyrrolidinone, and water. Specifically, in order to determine the amount of urine remaining in the bladder after spinal cord injury, bladder extrusion was performed twice a day by applying pressure to the central bottom portion of the lower abdomen using the index and middle fingers to empty the bladder, and the expelled urine was collected in a catheter and its volume was recorded. In addition, the UBFS was assessed daily using a 0-4 point UBFS (0 = total loss of function (manual bladder extrusion 3 times a day, bloody urine), 1 = partial restoration of UBF (manual extrusion 2 times a day, bloody urine), 2 = partial restoration of UBF (partial/complete release of sphincter spasm, manual extrusion once a day, persistently bloody urine), 3 = high restoration (manual extrusion once a day, no bloody urine), and 4 = normal UBF) as in the reference (Zeng et al., Spinal Cord (2017) 55: 834-839).

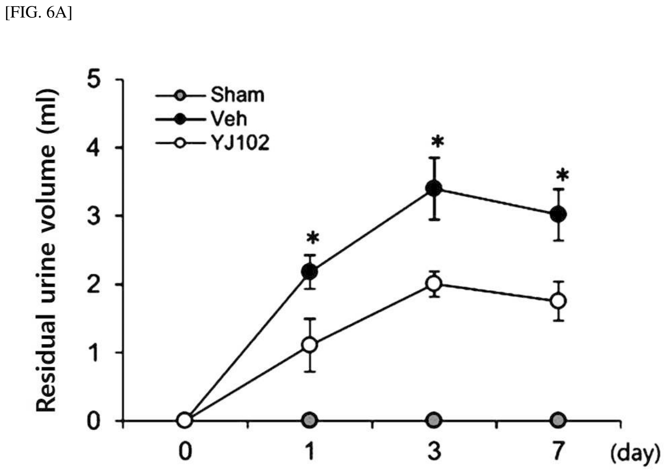

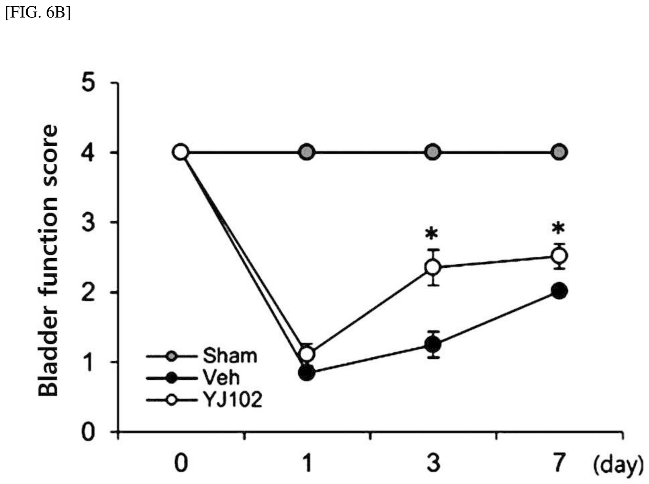

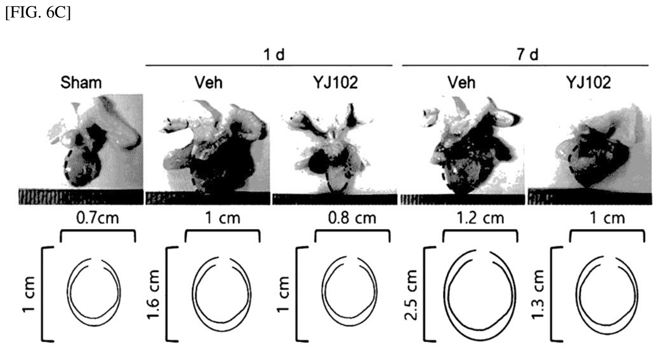

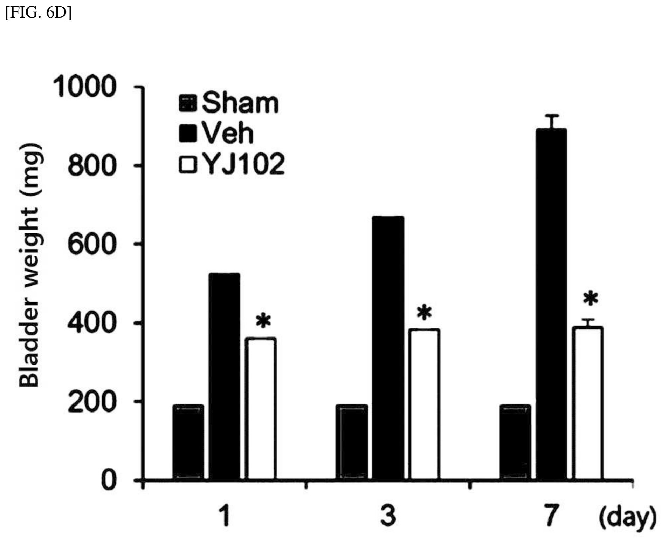

As a result, in the case of normal rats (Sham group), there was little amount of urine in the bladder because the rats excreted the urine by themselves, whereas in a Vehicle group, which was unable to excrete the urine by themselves after spinal cord injury, the amount of remaining urine was significantly increased compared to that of the Sham group. However, in an YJ102 administered group, the residual urine volume was significantly reduced compared to the Veh group from the first day of administration (FIG. 6A). In addition, as a result of the bladder function restoration analysis using the UBFS, it was shown that in the YJ102 administered group, the residual urine volume was significantly increased compared to the Vehicle group on day 3 and day 7 after spinal cord injury (FIG. 6B). Further, the bladder size and weight were also significantly reduced by YJ102 administration (FIGS. 6C and 6D).

### 4-2. Analysis of bladder hypertrophy and changes in collagen content in bladder muscle

As one indicator of bladder dysfunction, bladder hypertrophy, which was a thicker bladder wall, and changes in collagen content in the bladder muscle were identified. Specifically, a spinal cord injury (SNI) animal model was perfused through the left ventricle, and fixed with 4% paraformaldehyde, and the bladder was extracted, and then embedded in an optimal cutting temperature (OCT) compound, and the tissue was sectioned at a 10 µm thickness using a cryostat. In order to measure the bladder wall thickness, Hematoxylene & Eosin (Abcam) staining was performed according to a manufacturer's protocol, and in order to measure the collagen content in the muscle, Masson's trichrome (Sigma-Aldrich) staining was performed.

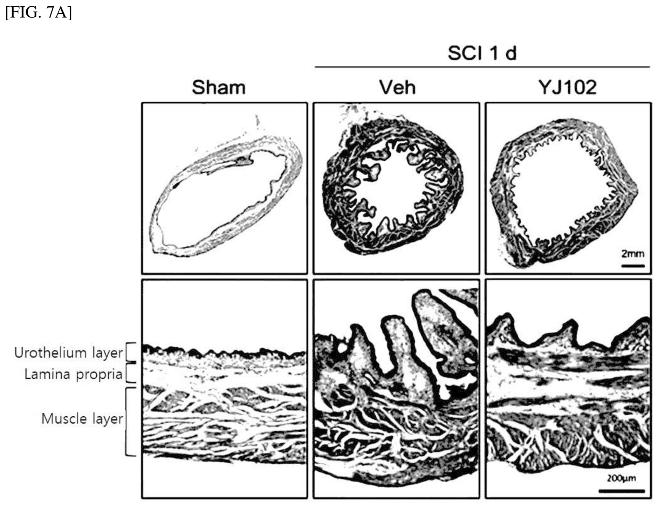

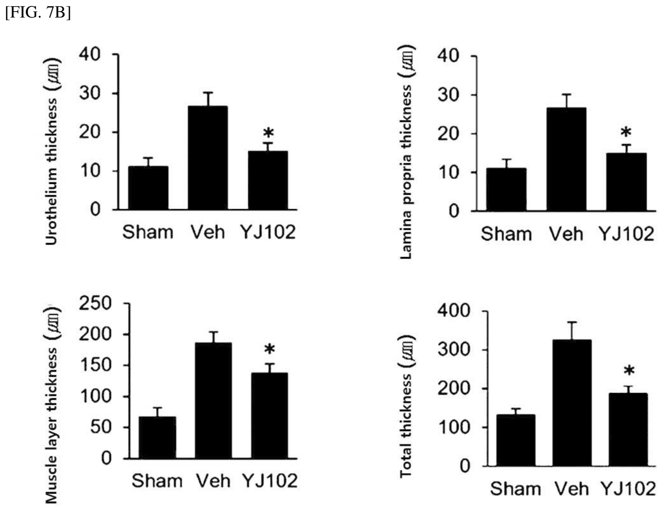

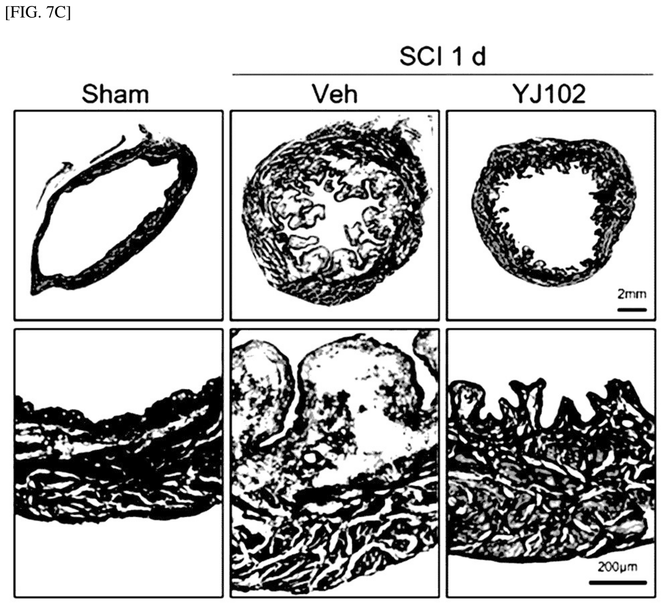

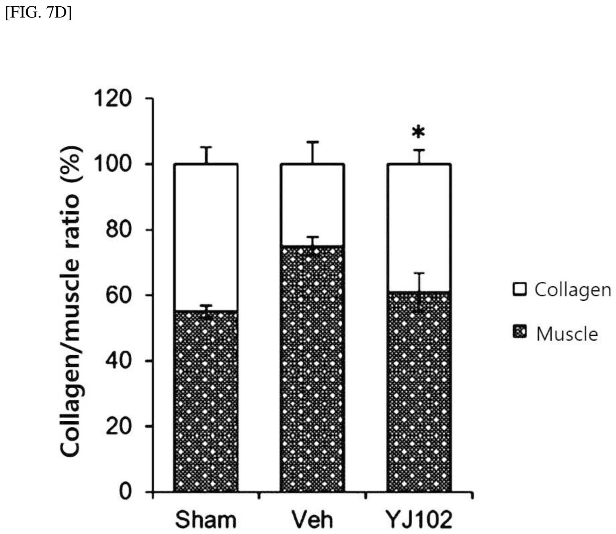

As a result, it was shown that the bladder wall was significantly thickened on day 1 after spinal cord injury, and the urothelium layer, lamina propria, and muscle layer constituting the bladder wall were increased in thickness by 2 to 3 times compared to a normal group. In contrast, in the YJ102 administered group, the thicknesses of the urothelium layer, lamina propria, and muscle layer were all significantly reduced (FIGS. 7A and 7B), and the reduced collagen content in the muscle after spinal cord injury was also maintained at a level almost similar to that of the normal group (FIGS. 7C and 7D).

### Example 5. Analysis of treatment mechanism for urinary dysfunction

### 5-1. Inhibition of urothelial tight junction protein loss

When bladder dysfunction occurred due to spinal cord injury, cystitis, etc., a decrease in tight junction protein in the urothelium in contact with urine occurred, which increased the permeability of the bladder wall to introduce the harmful substances into the tissue, and caused the death of the urothelium serving as a barrier, and an increase in inflammatory response in the bladder. In order to determine what effect the compound YJ102 of the present disclosure had on these pathological processes, the expression of tight junction proteins ZO-1 and occludin was confirmed by Western blot analysis and immunofluorescence staining, and the introduction of neutrophils and macrophages into the bladder was confirmed by immunofluorescence staining. Specifically, the cut bladder tissue sections were frozen in liquid nitrogen, ground in a mortar and pestle to be completely lysed, and homogenized by adding Lysis Buffer [50 mM Tris-HCl (pH 7.4), 150 mM NaCl, 2 mM MgCl₂, 0.1% NP40]. The homogenized tissue sections were transferred to a microcentrifuge tube, rock at 4°C for 20 minutes, and then centrifuged at 25,000 × g for 10 minutes. The supernatant was transferred to a new microcentrifuge, the protein concentrations were measured using a BCA kit, and then samples for Western blot were prepared by adding a 1X sample buffer and boiling for 5 minutes. For Western blot, the same amounts of proteins were loaded onto SDS-PAGE, electrophored, and then transferred to a nitrocellulose membrane, reacted with primary antibodies Occludin (Invitrogen), ZO-1 (Invitrogen), ED-1 (Serotec), and β-actin (Sigma), and then blotted with secondary antibodies. In addition, for immunofluorescence staining, 10 µm-thick bladder tissue sections were treated with primary antibodies ZO-1 (Invitrogen), Occludin (Invitrogen), MPO (DAKO), and ED-1 (Serotec), respectively, and reacted according to a manufacturer's protocol, and then color-developed with secondary antibodies (Jackson immunoresearh).

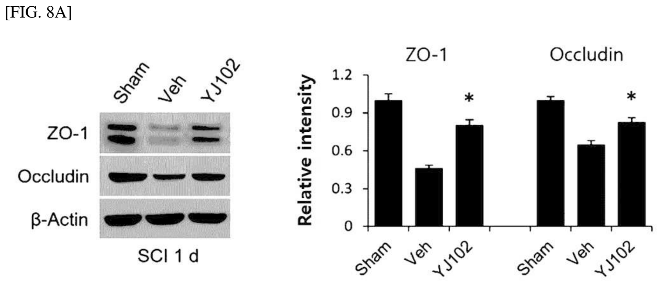

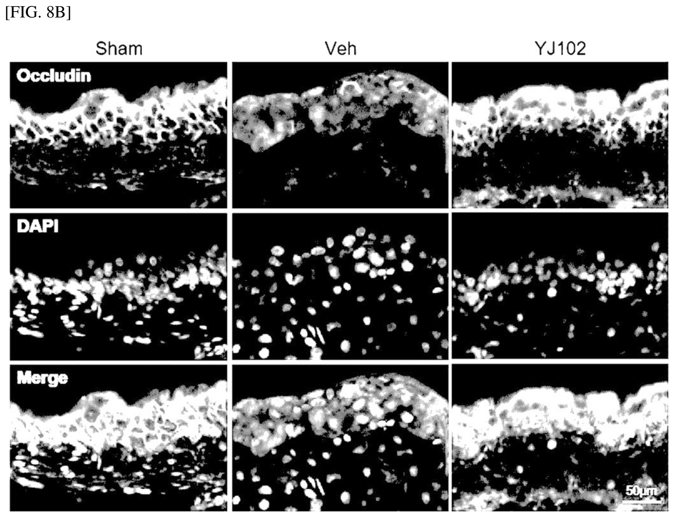

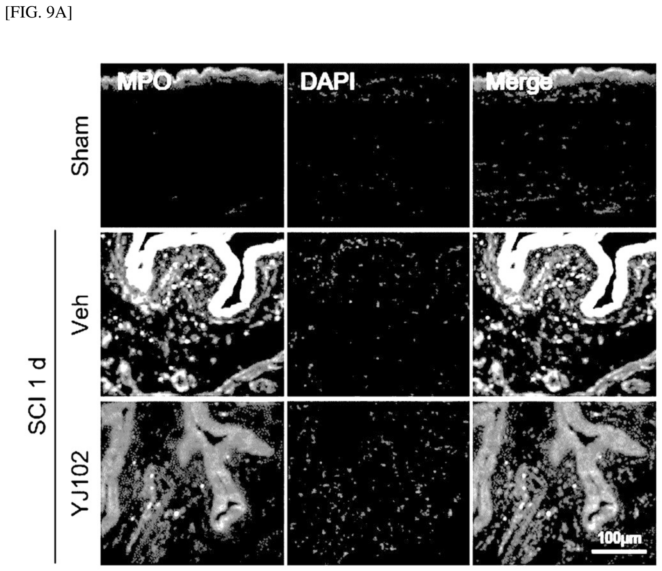

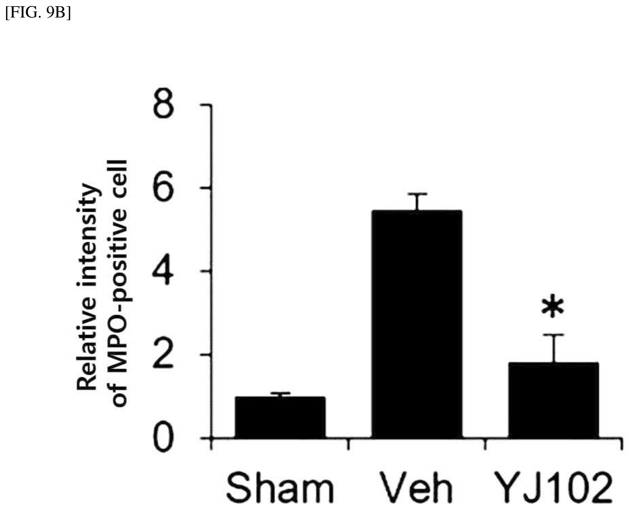

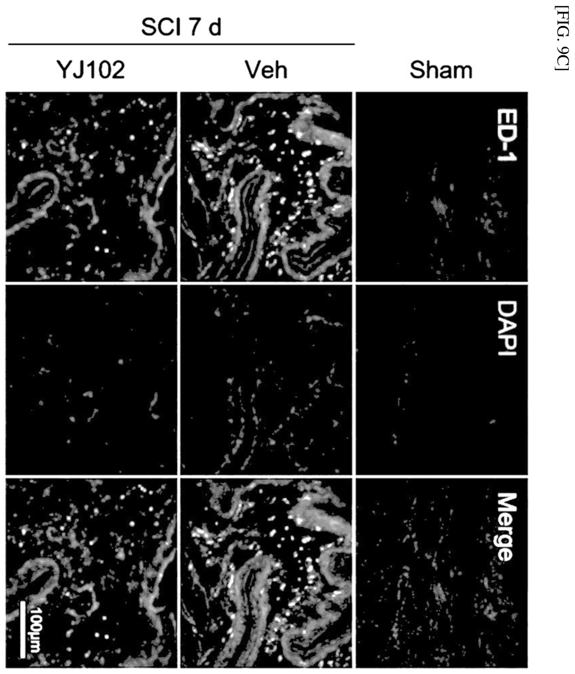

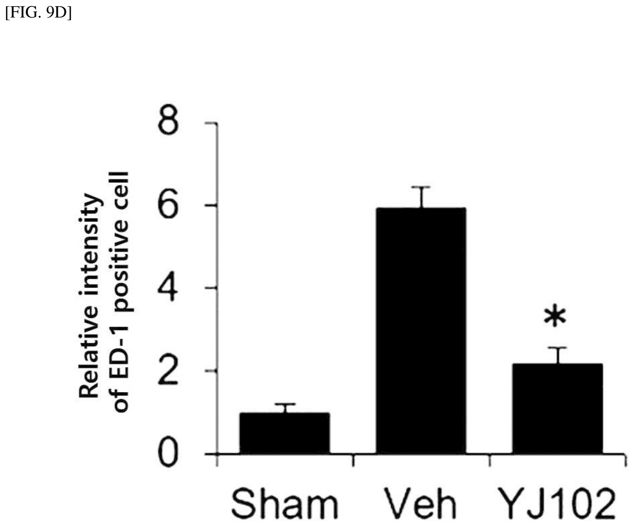

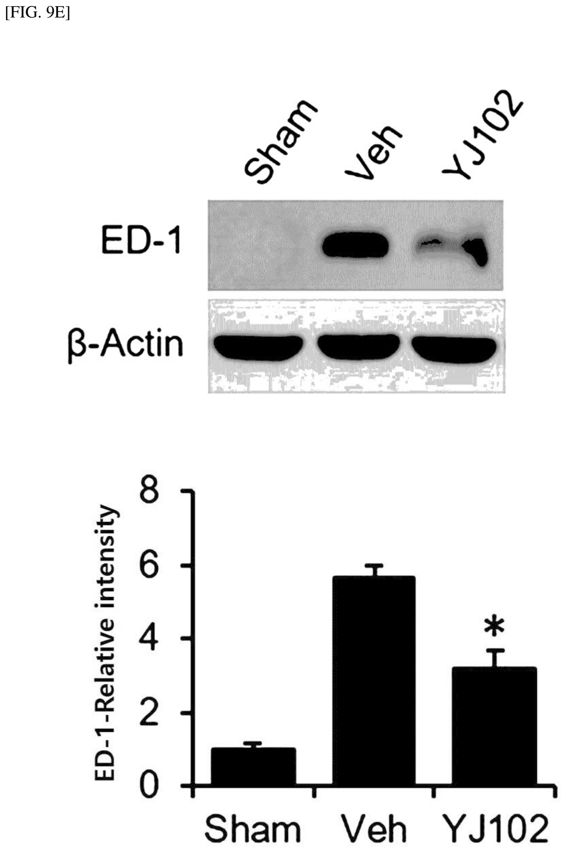

As a result, the amounts of ZO-1 and Occludin were significantly reduced at day 1 after spinal cord injury (SCI 1d), which was significantly inhibited by YJ102 treatment (FIG. 8A). Even as the mmunofluorescence staining results, it was shown that the expression of occludin in the urothelium was significantly reduced after spinal cord injury, and the reduction in Occludin was inhibited by YJ102 treatment (FIG. 8B). In addition, neutrophils and macrophages were identified in bladder tissue, and as a result, it was confirmed that the introduction of MPO-positive neutrophils and ED-1-positive macrophages into the bladder was significantly inhibited by YJ102 after spinal cord injury (FIG. 9). As a result, it was found that YJ102 protected the tight junction proteins in the urothelium and blocked the introduction of immune cells into the bladder.

### 5-2. Inhibition of p75 mediated-urothelial cell apoptosis

The effect of YJ102 on urothelial cell apoptosis in bladder tissue after spinal cord injury was confirmed by TUNEL staining, and the protein levels of cleaved caspase-3 indicating caspase-3 activation, and UPIII as a urothelial marker were confirmed by Western blot analysis using p75 (Promega), cleaved caspase-3 (Cell signaling technology), and UPIII (Abcam) as primary antibodies. In addition, in order to confirm whether the mechanism of apoptosis was caused by a p75 neurotrophin receptor, which was involved in urothelial apoptosis, the expression level of p75 was confirmed by RT-PCR, Western blot analysis, and immunofluorescence staining. For RT-PCR analysis, the obtained bladder tissue sections were frozen in liquid nitrogen, and ground in a mortar and pestle to be completely lysed, and then RNA was isolated using a TRIZOL Reagent (Invitrogen) according to a manual, and 20 µ1 of the total mixture was prepared by using 1 µg of total RNA, and cDNA was synthesized using MMLV reverse transcriptase (Invitrogen). The gene expression of p75 was analyzed using a cDNA template and primers shown in Table 2 below.

**[Table 2]**

| **Target** | **Primer** | **Sequence** |
|---|---|---|
| NGF | Forward | 5'-CAGGCAGAACCGTACACAGA-3' |
| | Reverse | 5'-GTCCGAAGAGGTGGGTGGAG-3' |
| p75 | Forward | 5'-AGCCAACCAGACCGTGTGTG-3' |
| | Reverse | 5'-TTGCAGCTGTTCCACCTCTT-3' |
| MMP-2 | Forward | 5'-ACCGTCGCCCATCATCAA-3' |
| | Reverse | 5'-TTGC ACTGCCAACTCTTTGTCT-3' |
| MMP-9 | Forward | 5'-TCGAAGGCG ACCTCAAGTG-3' |
| | Reverse | 5'-TTCGGTGTAGCTT TGGATCCA-3' |
| Abcc8 | Forward | 5'-CCTGCAGCCAGACATAGACA -3' |
| | Reverse | 5'-CAGTCAGCATGAGGCAGGTA-3' |
| TrpM4 | Forward | 5'-CAGCGACCTCTACTGGAAGG-3' |
| | Reverse | 5'-TCACGAGCTTGTGCCAATAG-3' |
| ZO-1 | Forward | 5'-AAGGCAATTCCGTATCGTTG-3' |
| | Reverse | 5'-CCACAGCTGAAGGACTCACA-3' |
| occludin | Forward | 5'-GCCTTTTGCTTCATCGCTTCC-3' |
| | Reverse | 5'-AACAATGATTAAAGCAAAAGCCAC-3' |
| IL-1β | Forward | 5'-GCAGCTACCTATGTCTTGCCCGTG-3' |
| | Reverse | 5'-GTCGTTGCTTGTCTCTCCTTGTA-3' |
| TNF-α | Forward | 5'-CCCAGACCCTCACACTCAGAT-3' |
| | Reverse | 5'-TTGTCCCTTGAAGAGAACCTG-3' |
| COX-2 | Forward | 5'-CCATGTCAAAACCGTGGTGAATG-3' |
| | Reverse | 5'-ATGGGAGTTGGGCAGTCATCAG-3' |
| iNOS | Forward | 5'-CTCCATGACTCTCAGCACAGAG-3' |
| | Reverse | 5'-GCACCGAAGATATCCTCATGAT-3' |
| MCP-1 | Forward | 5'-TCAGCCAGATGCAGTTAACG-3' |
| | Reverse | 5'-GATCCTCTTGTAGCTCTCCAGC-3' |
| MIP-1α | Forward | 5'-ACTGCCTGCTGCTTCTCCTACA-3' |
| | Reverse | 5'-AGGAAAATGACACCTGGCTGG-3' |
| MIP-1β | Forward | 5'-TCCCACTTCCTGCTGTTTCTCT-3' |
| | Reverse | 5'-GAATACCACAGCTGGCTTGGA-3' |
| Gro-α | Forward | 5'-CCGAAGTCATAGCCACACTCAA-3' |
| | Reverse | 5'-GCAGTCGTCTCTTTCTCCGTTAC-3' |
| MIP-2α | Forward | 5'-AGACAGAAGTCATAGCCACTCTCAAG-3' |
| | Reverse | 5'-CCTCCTTTCCAGGTCAGTTAGC-3' |
| GAPDH | Forward | 5'-AACTTTGGCATTGTGGAAGG-3' |
| | Reverse | 5'-GGAGACAACCAGGTCCTCAG' |

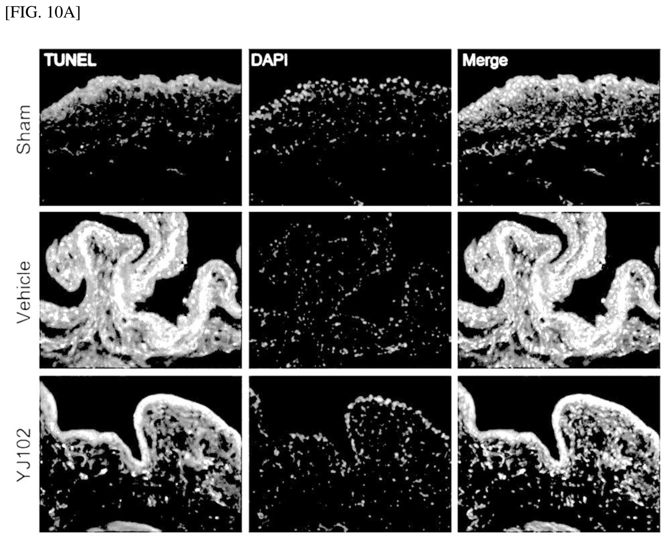

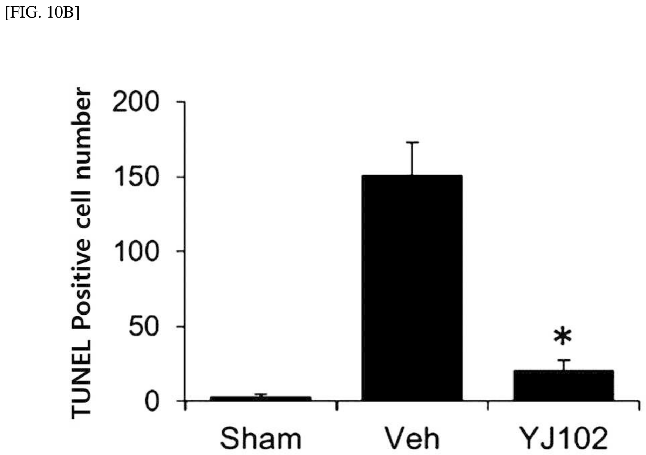

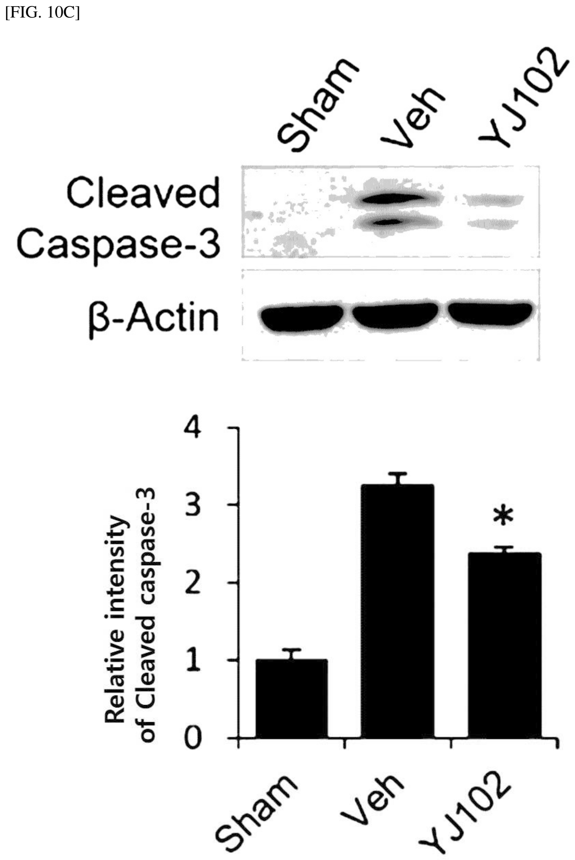

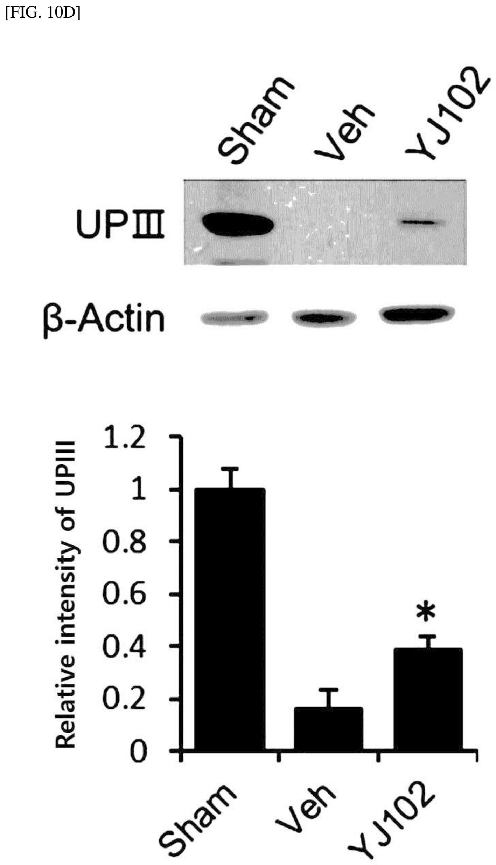

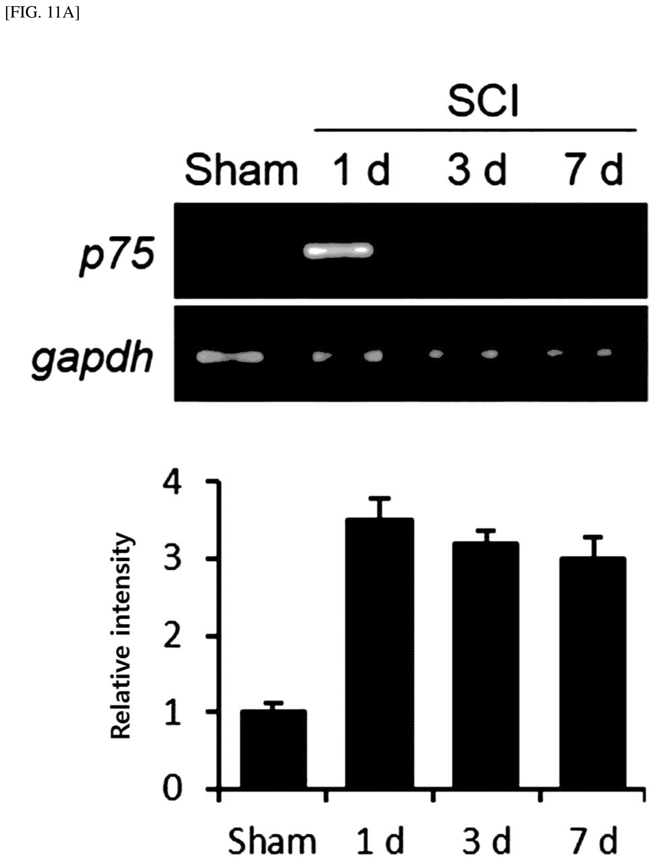

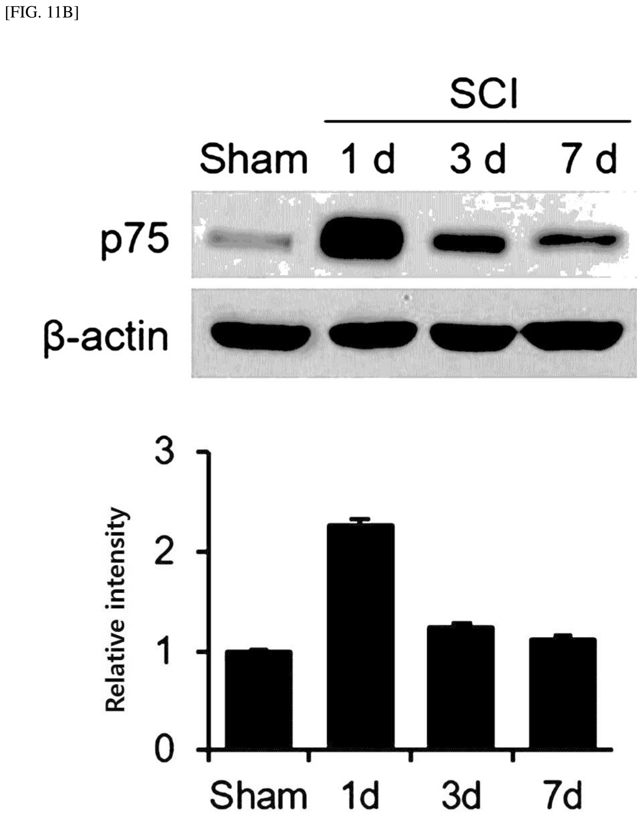

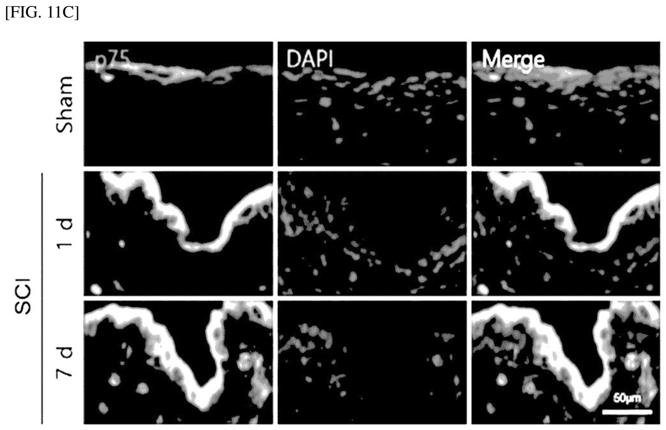

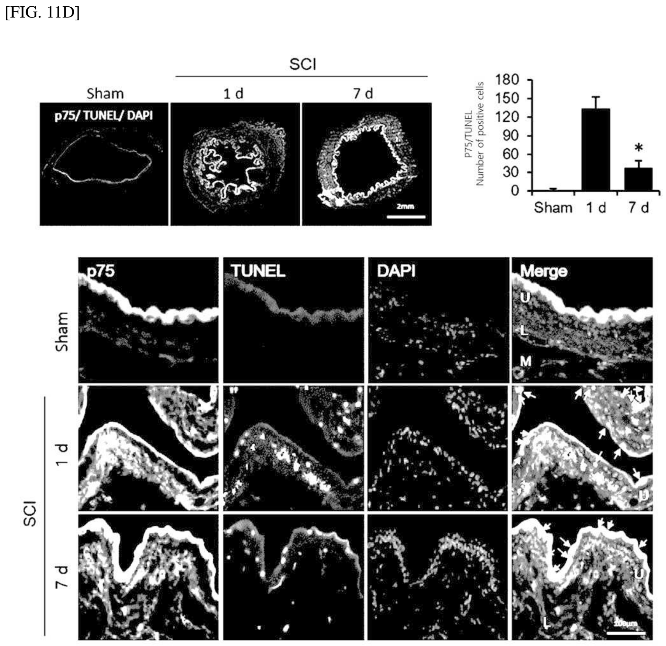

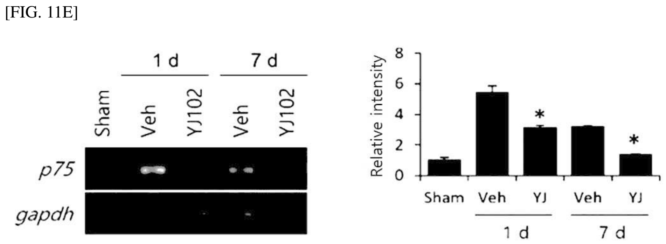

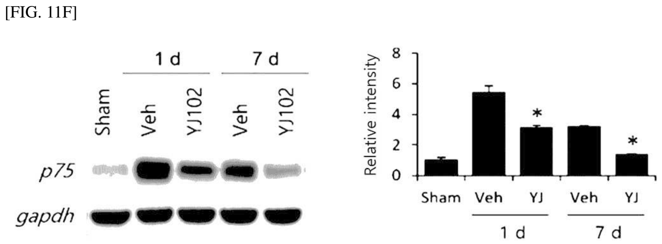

As the TUNEL analysis result, it was shown that the number of TUNEL-positive cells in the urothelium was significantly reduced in the YJ102 administered group compared to the Vehicle group (FIGS. 10A and 10B), and caspase-3 activation was also inhibited by YJ102 (FIG. 10C). In addition, it was shown that the level of UPIII as a urothelial marker was significantly reduced after spinal cord injury (meaning urothelial loss), which was inhibited by YJ102 administration (FIG. 10D). In addition, it was confirmed that the gene (mRNA) and protein expression of p75 were significantly increased after spinal cord injury (FIGS. 11A to 11C), and the urothelium expressing p75 was TUNEL-positive, and thus it was confirmed that p75 was mediated in the apoptosis process of the urothelium (FIG. 11D). It was shown that the increased p75 expression was significantly reduced by YJ102 treatment (FIGS. 11E and 11F).

As a result, it was confirmed that YJ102 had a cytoprotective effect that inhibited urothelial apoptosis, which was mediated through p75 receptor signaling.

### 5-3. Inhibition of proNGF expression

In order to determine whether YJ102 affected the production of proNGF as a ligand of p75, the expression of proNGF in the bladder was confirmed by RT-PCR, Western blot analysis, and immunofluorescence staining. In addition, for further verification, a macrophage cell line Raw 264.7 (Korea Cell Line Bank, Seoul, Korea) was stimulated with 1 µg/ml of LPS, and the effect of YJ102 on the production of proNGF was investigated. At this time, YJ102 was dissolved in DMSO and treated 30 minutes before LPS treatment.

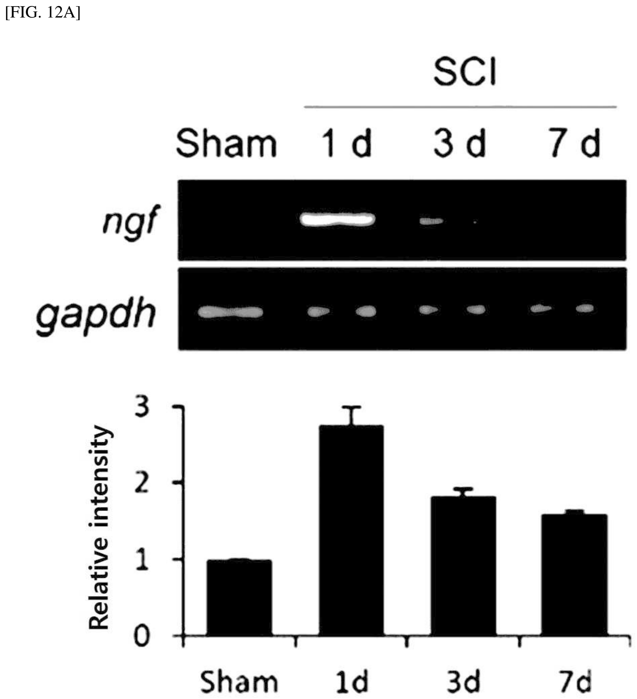

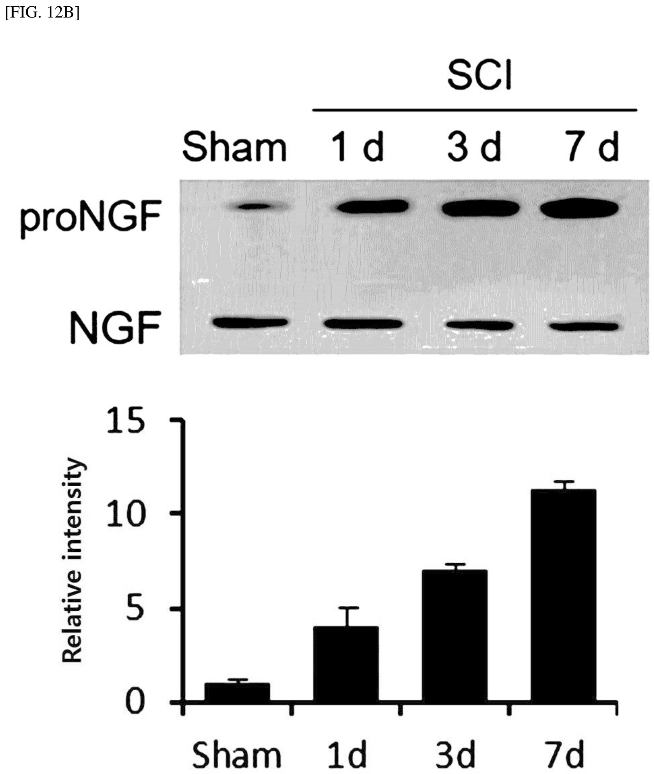

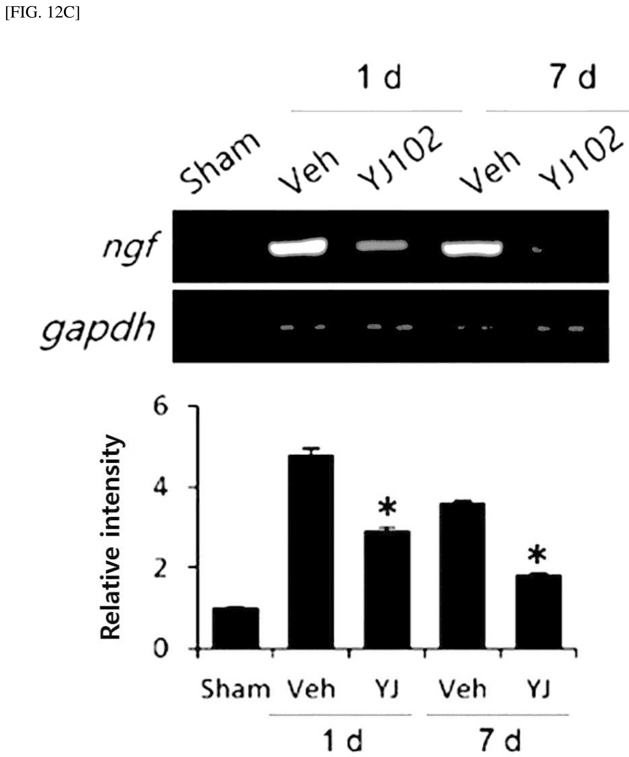

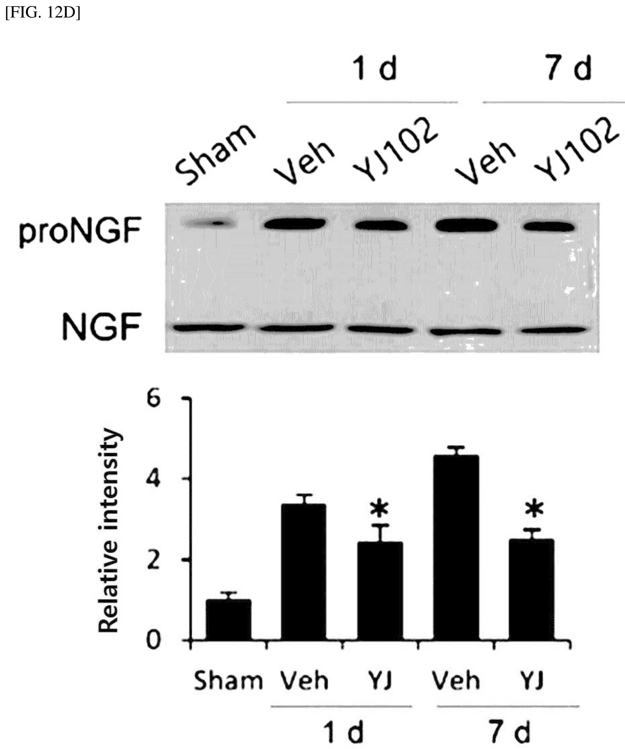

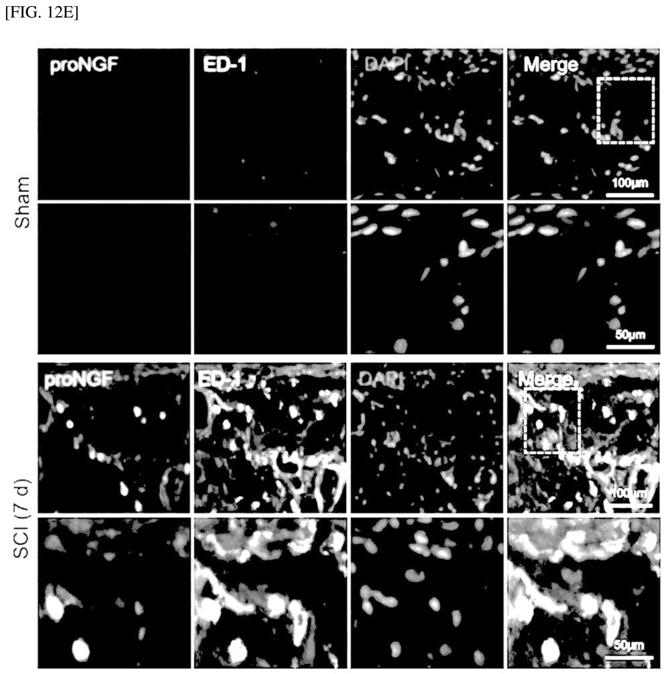

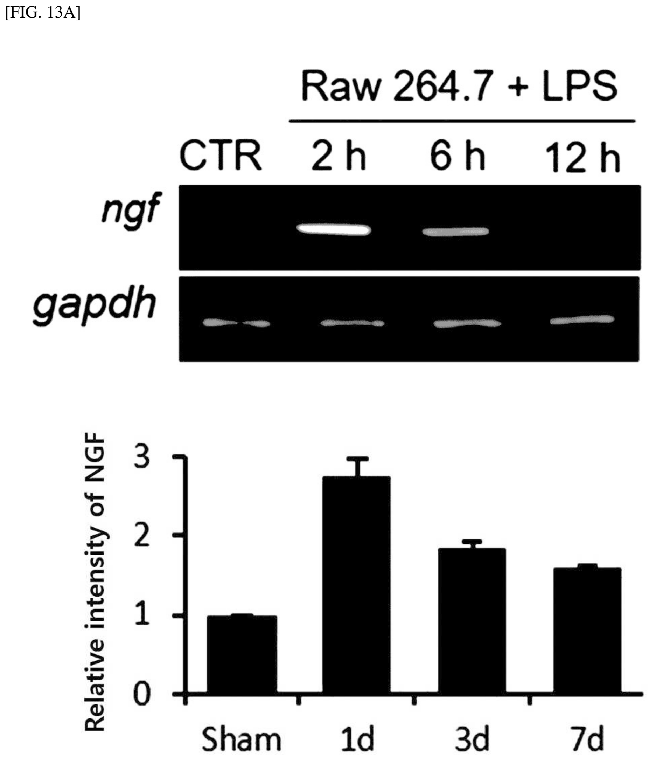

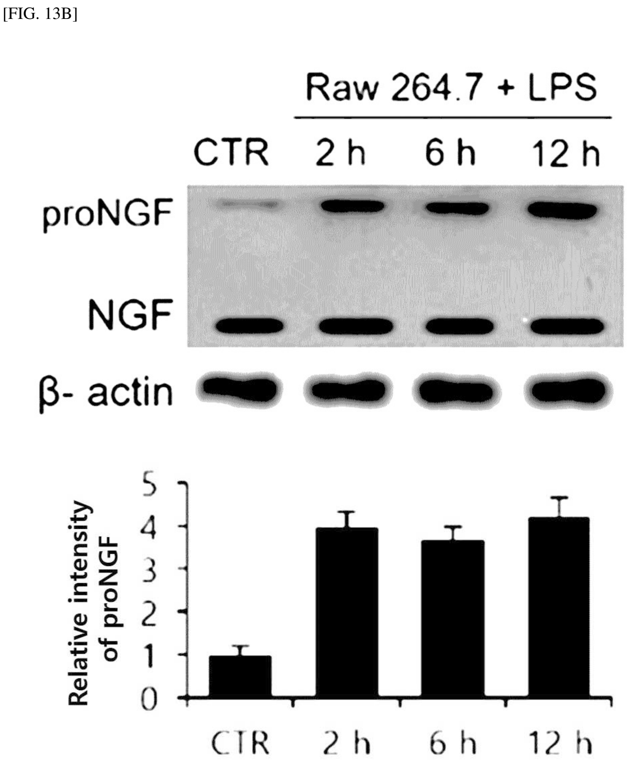

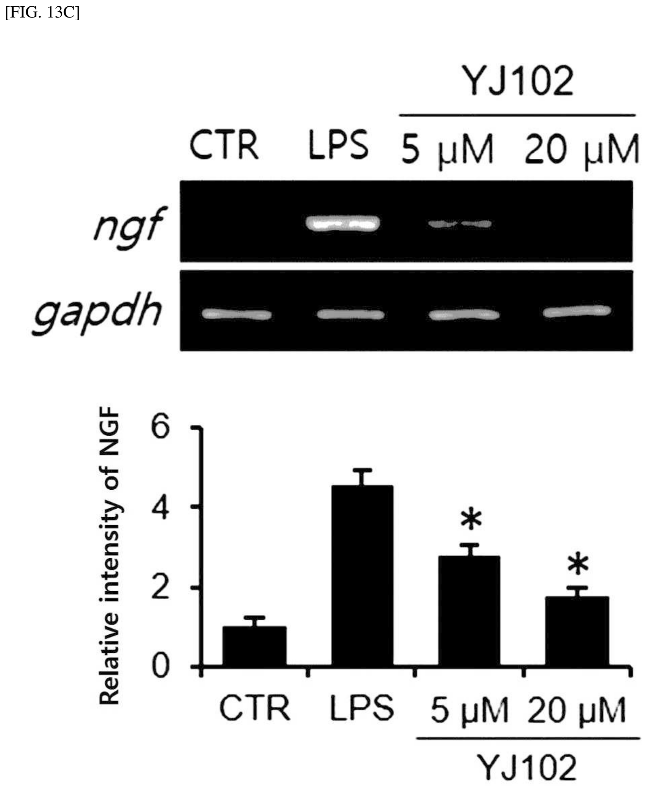

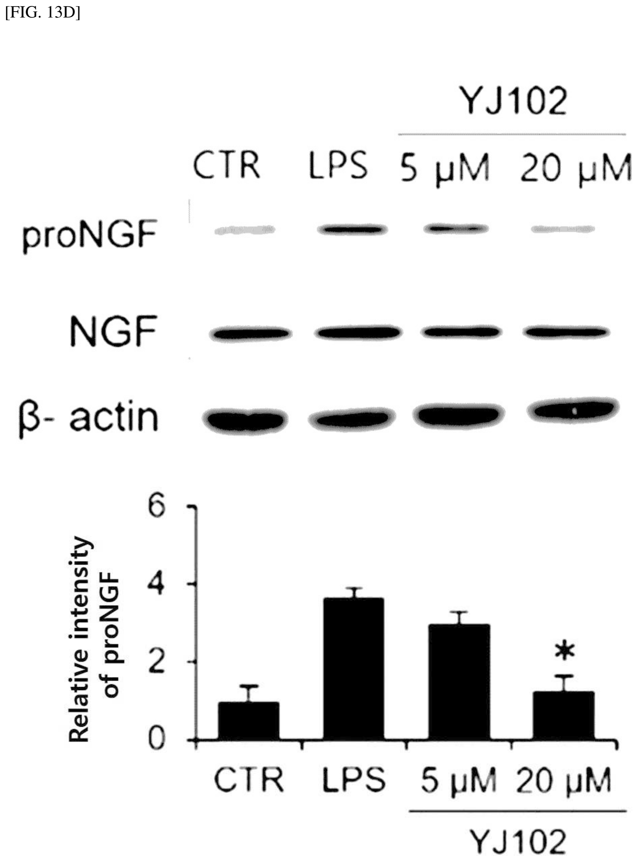

As a result, it was confirmed that YJ102 significantly inhibited NGF mRNA expression and proNGF production in bladder tissue after spinal cord injury (FIGS. 12A to 12D), and proNGF was expressed in macrophages (ED-1 positive cells) introduced into the bladder (FIG. 12E). As the result confirmed in the macrophage cell line, it was also shown that YJ102 significantly inhibited the mRNA and protein expression of proNGF (FIG. 13).

As a result, it was meant that YJ102 had a cytoprotective effect of inhibiting the urothelial apoptosis by inhibiting the expression of p75 and its ligand, proNGF, in the urothelium of the bladder after spinal cord injury.

### 5-4. Inhibition of inflammatory factors

To confirm the effect of YJ102 on the expression of inflammatory factors in the bladder after spinal cord injury, the expression of inflammatory cytokines and chemokines, COX-2, iNOS, IL-6, IL-1β, MIP-1α, MIP-1β, MIP-2α, and Gro-α in bladder tissue was confirmed by RT-PCR analysis and Western blot analysis using the primers in Table 2 above. In addition, even in the Raw 264.7 cell line, the expression was verified by RT-PCR analysis.

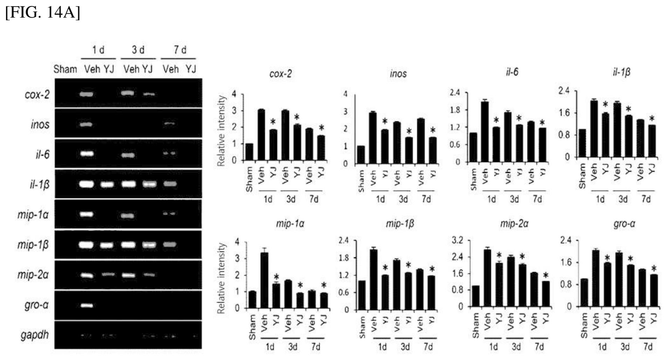

As a result, it was found that the expression of COX-2, iNOS, IL-6, IL-1β, MIP-1α, MIP-1β, MIP-2α, and Gro-α was all significantly reduced by YJ102 administration (FIG. 14), and it was confirmed that even in the macrophage cell line, the mRNA levels of IL-1β, TNF-α, IL-6, iNOS, and COX-2, whose expression was increased by LPS treatment, were significantly reduced by YJ102 treatment (FIG. 15).

As a result, it was confirmed that after traumatic spinal cord injury, YJ102 may restore bladder functions by reducing urothelial cell apoptosis, tight junction breakdown, cell infiltration, and resulting inflammatory response in the bladder, and inhibiting deformation of the bladder tissue.

### Example 6. Preparation of psoriasis animal model

Psoriasis caused by excessive production of skin cells was a chronic skin disease associated with various factors, including autoimmune diseases, and its symptoms included red and thickened skin and abnormally occurring scaling. As such, in order to prepare a disease animal model of psoriasis that was exhibited as an important clinically similar disease marker, such as epidermal hyperplasia, immune cell infiltration in the dermis and epidermis, dyskeratosis, inflammatory response, and activation of the IL-23/IL-17 cytokine axis of the skin, imiquimod (IMQ) was administered to mice. Specifically, the back region of a C57BL/6 mouse (male, 18 to 20 g, 8 weeks) (Daehan Biolink, Eumseong, Korea) was shaved to remove hair, and then applied with IMQ cream (83 mg/day) (5%) for consecutive 5 days from Day 0 to Day 4 to induce psoriasis-like skin inflammation, and a normal control mouse (Sham) was applied with Vaseline cream (FIG. 16A).

### Example 7. Confirmation of psoriasis treatment effect of compound of the present disclosure

### 7-1. Alleviation of erythema and scaling

In order to confirm the effect of YJ102 (Chemical Formula 1), a compound of the present disclosure, on psoriasis, YJ102 dissolved in DMSO, methyl pyrrolidinone, and water (1 : 1.5 : 1) was administered intraperitoneally (5 mg/kg) for a total of 3 days on Day 2, Day 3, and Day 4, and the Vehicle group was administered in the same amount of a solution of DMSO, methyl pyrrolidinone, and water (1 : 1.5 : 1) (FIG. 16A). To assess daily skin changes, PASI scores were used for erythema and scaling. The PASI score was evaluated as a severity scale of 0 to 4, in which 0 meant none, 1 meant mild, 2 meant moderate, 3 meant severe, and 4 meant very severe. (PASI Reference: Yashpal Manchanda, Abhishek De, Sudip Das, and Disha Chakraborty. Disease Assessment in Psoriasis. Indian J Dermatol. 2023 May-Jun; 68 (3): 278-281.)

As a result, the psoriasis mouse model (Vehicle group) prepared in Example 1 above began to show erythema and scaling on the skin from Day 2 after applying IMQ, and the symptoms were worsened on Day 4 and Day 5, and then the erythema and scaling were observed over the entire back. On the other hand, in a group treated with YJ102, the degree of erythema and scaling was significantly lower than that of the Vehicle group, and the symptoms appeared to be alleviated on Day 6 (FIGS. 16B to 16D).

### 7-2. Inhibition of increase in epidermal layer thickness

On day 6, the skin tissue of the psoriasis mouse model was stained with Hematoxyline & Eosin, and the epidermal layer thickness was measured. Specifically, on Day 6, the skin of the mouse back was extracted, immersed in 4% paraformaldehyde for 24 hours to fix the tissue, and then paraffin blocks were made to prepare paraffin tissue sections with a thickness of 5 µm. In order to measure the thickness of the epidermal layer, paraffin tissue was stained with Hematoxylene & Eosin (Abcam) according to the manufacturer's protocol, and five sections of the epidermal layer from each mouse were photographed at 200x magnification using an optical microscope. The epidermal layer thickness was measured in tissues from a total of five mice (a total of 25 sections) and the average value was calculated.

As a result, in the Sham group, which was a normal control group, the epidermal layer thickness was 36.6 ± 2.3 µm, and in the Vehicle group, which was a psoriasis-inducing group, the epidermal layer thickness was 141.3 ± 7.5 µm, which was approximately 38 times thicker than the Sham group. On the other hand, the epidermal layer thickness of the group administered with YJ102 was 89.8 ± 6.8 µm, which was reduced by approximately 35% compared to the Vehicle group. In addition, in the skin tissue of the Vehicle group, a significant number of cells introduced into the dermal layer was observed, but in the YJ102 administered group, the number of the cells was decreased to the Sham level (FIG. 17).

As a result, it was confirmed that YJ102 may alleviate the symptoms of psoriasis.

### Example 8. Confirmation of the inhibitory effect of compound of the present disclosure on spleen hypertrophy caused by psoriasis

The spleen hypertrophy occurred in mice induced with psoriasis, the longitudinal diameter of the spleen in psoriasis patients was increased compared to that of non-psoriasis patients, and a correlation between the disease duration and the spleen diameter was reported. Therefore, on Day 6, the spleen of the mouse in each group was extracted and weighed to determine the degree of spleen hypertrophy.

As a result, the size and weight of the spleen in the YJ102 group were significantly reduced compared to the Vehicle group (FIG. 18). Therefore, as the result showing that YJ102 reduced the spleen hypertrophy in the IMQ psoriasis animal model, it was suggested that YJ102 may have the potential to control immune system abnormalities in psoriasis patients.

## Claims

1. A pharmaceutical composition for preventing or treating neuropathic pain, neuroinflammation, bladder diseases, neurogenic bladder or inflammatory skin diseases, comprising a compound represented by Chemical Formula 1, an isomer thereof, a solvate thereof, a hydrate thereof, or a salt thereof as an active ingredient:

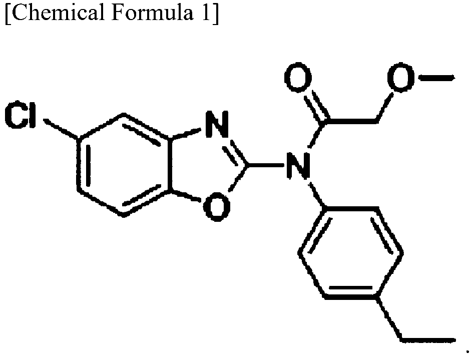

2. The pharmaceutical composition of claim 1, wherein the neuropathic pain includes pain caused by peripheral or central nerve injury or dysfunction.

3. The pharmaceutical composition of claim 1, wherein the neuropathic pain is diabetic peripheral neuropathic pain, central sensitization, shingles, postherpetic neuralgia, trigeminal neuralgia, complex regional pain syndrome, reflex sympathetic dystrophy, migraine, phantom limb pain, neuropathic pain caused by trauma, neuropathic pain caused by nerve injury, neuropathic pain caused by spinal stenosis, neuropathic pain caused by spinal cord injury, neuropathic pain caused by chronic diseases (multiple sclerosis, HIV, etc.), neuropathic pain caused by trauma (causalgia), neuropathic pain caused by impingement (i.e., sciatica, carpal tunnel, etc.), neuropathic pain caused by drug or toxic chemical exposure, neuropathic pain caused by infection or post-infection, neuropathic pain caused by damaged organ functions, neuropathic pain caused by vascular diseases, neuropathic pain caused by metabolic diseases, neuropathic pain caused by cancer or cancer treatment, neuropathic pain caused by autoimmune diseases, neuropathic back pain, neuropathic pain caused by fibromyalgia, or idiopathic neuropathic pain.

4. The pharmaceutical composition of claim 1, wherein the pharmaceutical composition inhibits the activation of microglia or astrocytes.

5. The pharmaceutical composition of claim 1, wherein the pharmaceutical composition inhibits the expression of p75, NGF, proNGF, Jumonji domain-containing protein 3 (JMJD3), transient receptor potential cation channel (TRPM7, subfamily M, member 7), IL-6, iNOS, COX-2, IL-1β, TNF-α, MIP-1α, MIP-1β, MIP-2α, Gro-α or MCP-1.

6. The pharmaceutical composition of claim 1, wherein the pharmaceutical composition inhibits phosphorylation of p38MAPK, JAK2, STAT3, S6 or p70S6 kinase.

7. The pharmaceutical composition of claim 1, wherein the pharmaceutical composition increases Trimethylated histone H3 at lysine 27 (H3K27Me3).

8. The pharmaceutical composition of claim 1, wherein the neuroinflammation includes neuroinflammation caused by the activation of microglia or astrocytes.

9. The pharmaceutical composition of claim 1, wherein the bladder diseases are urolithiasis, cystitis, prostate cancer, urinary incontinence, hematuria, bladder cancer, urethritis, prostatitis, urinary frequency, enuresis, urgency, oliguria, acute cystitis, hypospadias, urodynia, bladder stones, urethrostenosis, overactive bladder syndrome, chronic cystitis, urinary tract infections, genuine stress urinary incontinence, chronic prostatitis, urinary hesitancy, or chronic bladder failure.

10. The pharmaceutical composition of claim 9, wherein the bladder diseases are caused by spinal cord injury.

11. The pharmaceutical composition of claim 10, wherein the spinal cord injury is traumatic spinal cord injury, degenerative spinal diseases, inflammatory spinal diseases, spinal cord tumor, spinal cord malformation, spinal tumor, spinal cord hemorrhage, stroke, spinal cord paralysis caused by extramedullary vascular disorder, myelitis, multiple sclerosis, amyotrophic lateral sclerosis, or spinal cord stenosis.

12. The pharmaceutical composition of claim 1, wherein the neurogenic bladder is caused by spinal cord injury.

13. The pharmaceutical composition of claim 12, wherein the spinal cord injury is traumatic spinal cord injury, degenerative spinal diseases, inflammatory spinal diseases, spinal cord tumor, spinal cord malformation, spinal tumor, spinal cord hemorrhage, stroke, spinal cord paralysis caused by extramedullary vascular disorder, myelitis, multiple sclerosis, amyotrophic lateral sclerosis, or spinal cord stenosis.

14. The pharmaceutical composition of claim 1, wherein the neurogenic bladder exhibits symptoms of urinary retention, urinary urgency, urgency incontinence, urinary frequency, nocturia, dysuria, urinary disturbances, or pyelonephritis.

15. The pharmaceutical composition of claim 1, wherein the pharmaceutical composition alleviates spleen hypertrophy or bladder hypertrophy.

16. The pharmaceutical composition of claim 1, wherein the pharmaceutical composition increases the expression of ZO-1 and occludin in the urothelium.

17. The pharmaceutical composition of claim 1, wherein the pharmaceutical composition protects urothelial cells.

18. The pharmaceutical composition of claim 1, wherein the inflammatory skin diseases are autoimmune skin disease, a proliferative skin disease, fibrous skin disease, contact dermatitis, seborrheic dermatitis, scleroderma, skin immune hypersensitivity, atopic dermatitis, urticaria, pruritus, psoriasis or eczema.

19. The pharmaceutical composition of claim 18, wherein the psoriasis is guttate psoriasis, plaque psoriasis, erythrodermic psoriasis, pustular psoriasis and exfoliative psoriasis.

20. The pharmaceutical composition of claim 1, wherein the pharmaceutical composition inhibits or alleviates erythema, scaling or an increase in epidermal tissue thickness.

21. A composition for alleviating bladder dysfunction comprising a compound represented by Chemical Formula 1, an isomer thereof, a solvate thereof, a hydrate thereof, or a salt thereof as an active ingredient:

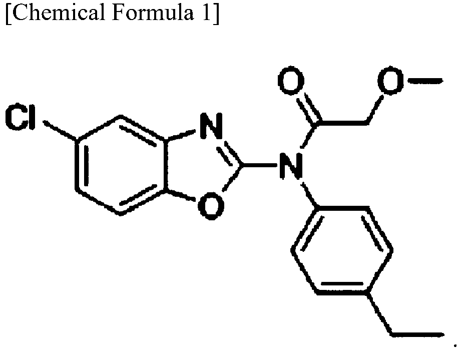

22. The composition for alleviating bladder dysfunction of claim 21, wherein the bladder dysfunction is bladder dysfunction caused by spinal cord injury.

23. The composition for alleviating bladder dysfunction of claim 22, wherein the spinal cord injury is traumatic spinal cord injury, degenerative spinal diseases, inflammatory spinal diseases, spinal cord tumor, spinal cord malformation, spinal tumor, spinal cord hemorrhage, stroke, spinal cord paralysis caused by extramedullary vascular disorder, myelitis, multiple sclerosis, amyotrophic lateral sclerosis, or spinal cord stenosis.

24. The composition for alleviating bladder dysfunction of claim 21, wherein the composition improves or restores the urinary functions of the bladder.

25. A composition for inhibiting bladder hypertrophy comprising a compound represented by Chemical Formula 1, an isomer thereof, a solvate thereof, a hydrate thereof, or a salt thereof as an active ingredient:

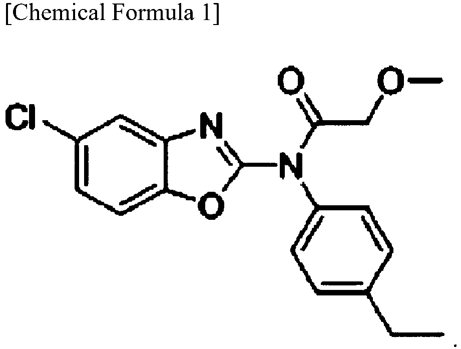

26. The composition for inhibiting bladder hypertrophy of claim 25, wherein the composition inhibits an increase in thickness of the urothelium layer, lamina propria, or muscle layer of the bladder.

27. A cosmetic composition for preventing or alleviating inflammatory skin diseases, comprising a compound represented by Chemical Formula 1, an isomer thereof, a solvate thereof, a hydrate thereof, or a salt thereof as an active ingredient:

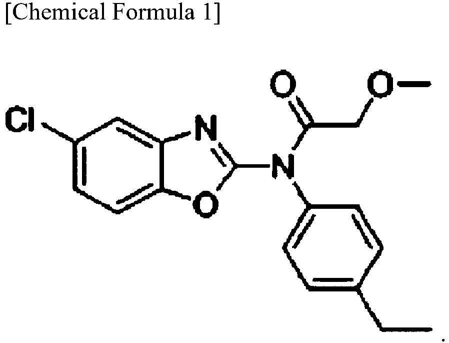

28. The cosmetic composition for preventing or alleviating inflammatory skin diseases of claim 27, wherein the cosmetic composition is prepared by at least one formulation selected from the group consisting of skin, lotion, cream, essence, emulsion, gel, hand cream, lipstick, cleansing foam, cleansing cream, cleansing water, spray, shampoo, rinse, treatment, body cleanser, soap, pack, massage agents, face powder, compact, foundation, two-way cake, and makeup base.

29. A use of a compound represented by Chemical Formula 1, an isomer thereof, a solvate thereof, a hydrate thereof, or a salt thereof, for the preparation of a pharmaceutical composition for preventing or treating neuropathic pain, neuroinflammation, bladder diseases, neurogenic bladder or inflammatory skin diseases:

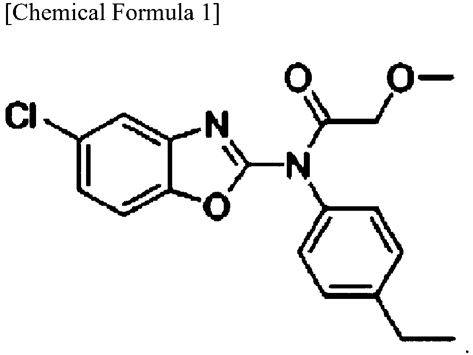

30. A use of a compound represented by Chemical Formula 1, an isomer thereof, a solvate thereof, a hydrate thereof, or a salt thereof, for the preparation of a composition for alleviating bladder dysfunction or inhibiting bladder hypertrophy:

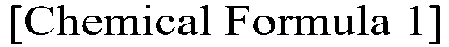

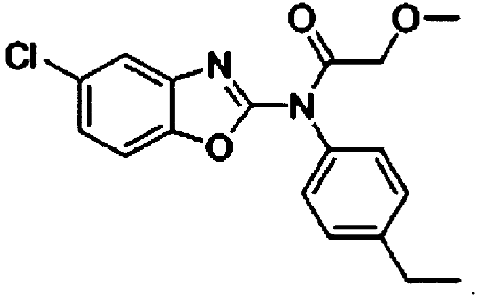

31. A method for treating neuropathic pain, neuroinflammation, bladder diseases, neurogenic bladder or inflammatory skin diseases, comprising administering a compound represented by Chemical Formula 1, an isomer thereof, a solvate thereof, a hydrate thereof, or a salt thereof to a subject suffering from neuropathic pain, neuroinflammation, bladder diseases, neurogenic bladder or inflammatory skin diseases:

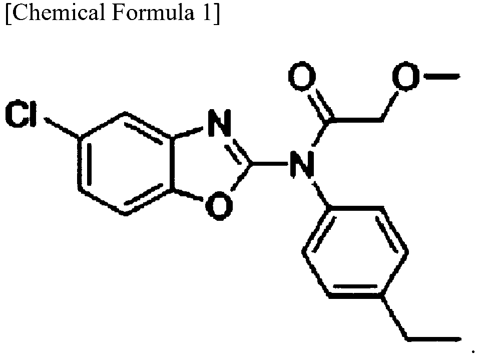

32. A method for treating or alleviating bladder dysfunction or bladder hypertrophy, comprising administering a compound represented by Chemical Formula 1, an isomer thereof, a solvate thereof, a hydrate thereof, or a salt thereof to a subject having symptoms of bladder dysfunction or bladder hypertrophy.
